(19) Europäisches Patentamt
European Patent Office
Office européen des brevets

(11) EP 2 530 072 A1

(12) **EUROPEAN PATENT APPLICATION**

(43) Date of publication:
**05.12.2012 Bulletin 2012/49**

(21) Application number: **11382184.7**

(22) Date of filing: **03.06.2011**

(51) Int Cl.:
***C07C 271/54*** *(2006.01)* ***A61K 31/27*** *(2006.01)*
***A61P 25/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **LACER, S.A.**
**08025 Barcelona (ES)**

(72) Inventors:
- **Mourelle Mancini, Marisabel**
  **E-08028 BARCELONA (ES)**
- **Del Castillo Nieto, Juan Carlos**
  **E-08025 BARCELONA (ES)**

(74) Representative: **ABG Patentes, S.L.**
**Avenida de Burgos, 16D**
**Edificio Euromor**
**28036 Madrid (ES)**

(54) **New compounds, synthesis and use thereof in the treatment of pain**

(57) The present invention relates to a compound of formula (I), or pharmaceutically acceptable, stereoisomers, salts or solvates thereof

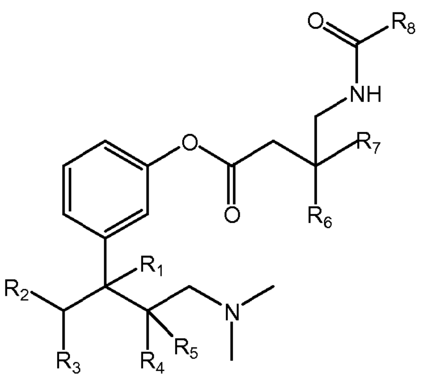

(I)

to methods for its synthesis, and use in the treatment of pain.

EP 2 530 072 A1

## Description

## FIELD OF THE INVENTION

The present invention relates to new compounds, their use for the treatment of pain, and synthesis thereof.

## BACKGROUND OF THE INVENTION

Chronic pain is a common problem that presents a major challenge to healthcare providers because of its complex natural history, unclear etiology, and poor response to therapy. The pathophysiology of chronic pain is multifactorial and complex and still is poorly understood. Some have even suggested that chronic pain might be a learned behavioral syndrome that begins with a noxious stimulus that causes pain. Patients with several psychological syndromes (e.g., major depression, somatization disorder, hypochondriasis, conversion disorder) are prone to developing chronic pain. Pain is the most common complaint that leads patients to seek medical care, and chronic pain is not uncommon. Approximately 35% of Americans have some element of chronic pain, and approximately 50 million Americans are disabled partially or totally due to chronic pain.

Various neuromuscular, reproductive, gastrointestinal, and urologic disorders may cause or contribute to chronic pain. Sometimes multiple contributing factors may be present in a single patient. The modem concept of pain treatment emphasizes the significance of prophylactic prevention of pain, as pain is more easily prevented than it is relieved. Pain is generally controlled by the administration of short acting analgesic agents, steroids and non-steroidal anti -inflammatory drugs. Analgesic agents include opiates, tricyclic antidepressants, anticonvulsivants, selective inhibitors of serotonin or noradrenalin reuptake and anti-inflammatory agents. A variety of active ingredients are available for the treatment of pain. As would be appreciated by the skilled person, therapeutics vary in pharmacological profile and effectiveness, and a large number of compounds exist which may be used for the treatment of pain, only effective in providing at most approximately a 35-50% reduction in pain.

Unfortunately, several adverse side effects are associated with the above mentioned treatments. Side effects may include, e.g., dizziness, somnolence, and harmful side effects are associated with most traditional analgesics at dosages effective for treating a neuropathy. As would be appreciated by the skill person, these side effects can include respiratory depression, disturbed sleep patterns, diminished appetite, seizures, and psychological and/or physical dependency.

For example, the pharmacological management of acute postoperative pain and chronic pain syndromes has been traditionally based on various regimens of opiates and their congeners or NSAIDs. All opiates have side effects, of which the most dangerous are respiratory and cardiovascular depression associated with excessive sedation. NSAIDs may also induce side effects such as exacerbation of bleeding tendencies and the impairment renal function.

Accordingly, there is a need to provide methods and compositions for the treatment of acute or chronic pain which provide effective control of pain with reduced harmful side effects. Clearly, there is a need for improved compositions and methods for the effective treatment of neuropathic pain.

Tramadol ((1R,2R)-rel-2-[(dimethylamino)methyl]-1-(3-methoxyphenyl) cyclohexanol) and tapentadol (3-[(1R, 2R)-3-(dimethylamino)-1-ethyl-2-methylpropyl] phenol hydrochloride) are two centrally-acting analgesics for treating moderate to moderately severe pain. Despite their usefulness, both compounds have drawbacks. Among the most relevant, is the fact that they are rapidly released in the gastrointestinal tract, but their analgesic activity rapidly fades, requiring administration at short intervals. None of them shows a sufficiently good behavior in the treatment of acute pain either.

Some efforts have been made to meet these problems of tramadol and tapentadol. WO 2009/067703 proposes a controlled released formulation of tapentadol further comprising as second active agent which is tramadol, a GABA analog or an NSAID (non-steroidal anti inflammatory drugs). For example, combinations are given of tapentadol with a second active ingredient such as gabapentin or pregabalin.

Further pharmaceutical compositions comprising tramadol or tapentadol and a second analgesic have been proposed. These seek to provide analgesic effect for an extended period of time and/or increase the analgesic effect itself. Thus, WO 2009/021058 discloses a pharmaceutical composition comprising a combination of three drugs, namely, tramadol or an analog thereof, an NMDA antagonist and gabapentin or an analog thereof. US 6,562,865 reports the synergistic effect of a combined administration of tramadol and gabapentin. This effect is however unclear, and could be exclusive of certain proportions between both drugs (E.E. Codd et al. Pain, 2008, 254-262) and of the method of administration (V. Granados-Soto et al. Pharmacology, 2005, 74, 200-208).

Further combinations have been proposed without fully overcoming the above mentioned problems. This is probably due to the complex interactions which take place between different drugs, wherein different factors have to be considered (e.g. proportions between drugs and method of administration).

A different approach has been the provision of derivatives of the tramadol analog, O-desmethyl tramadol

(3-[2-(1-Amino-1-methylethyl)-1-hydroxycyclohexyl] phenol), a known metabolite of tramadol which is even more active than tramadol itself. EP 1 251 120 Al and WO 00/27799 A1 provide esters of O-desmethyl tramadol or analogs thereof, wherein the acid moiety is an aromatic group. EP 0 753 506 provides further esters of O-desmethyl tramadol. US 2005/143355 further discloses esters of O-desmethyl tramadol wherein the acid moiety is an NSAID selected from ibuprofen, naproxen, indomethacin, diclofenac, dipyron, flurbiprofen, ketoprofen and acetylsalicylic acid. WO 2010/100477 A2 and CN 101845002 A provide carbamate derivatives of tapentadol, while EP 0 693 475 A1, EP 0 753 506 Al and WO 2004/108658 Al provide, among many others, (4-isopropyl-phenyl)-carbamoyl derivatives of a family of analgesic compounds comprising both tramadol and tapentadol.

Although the above compounds and compositions represent improvements in the treatment of pain, they still do not completely overcome the existing problems of tramadol or tapentadol therapies. There is still a need to provide further compounds with analgesic effect, which can substitute or complement existing therapies. Said compounds would preferably overcome the problems described above, and they would preferably show increased or synergistic analgesic activity with reduced toxicity or side effects and/or improved bioavailability.

## SUMMARY OF THE INVENTION

The present invention provides improved derivatives of O-desmethyl tramadol, tapentadol or analogs thereof, in the treatment of pain.

Accordingly, a first aspect of the invention is a compound of formula (I), or pharmaceutically acceptable, stereoisomers, salts or solvates thereof (compounds of the invention)

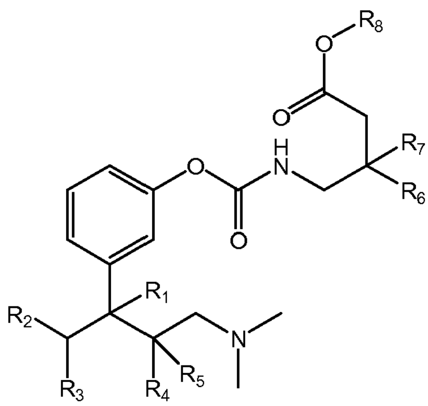

(I)

wherein
$R_1$ is selected from the group consisting of -H, and -OH;
$R_2$ is -H;
$R_5$ is -H;
or $R_1$ and $R_2$ together form a double bond and $R_5$ is -H
or $R_1$ and $R_5$ together form a double bond and $R_2$ is -H;
$R_3$ is a $C_1$-$C_6$ alkyl group;
$R_4$ is a $C_1$-$C_6$ alkyl group;
or $R_3$ and $R_4$ together form a $C_2$-$C_4$ alkylidene group;
$R_6$ is selected from the group consisting of -H and $C_1$-$C_6$ alkyl group;
$R_7$ is selected from the group consisting of -H and $C_1$-$C_6$ alkyl group;
or $R_6$ and $R_7$ together form a $C_4$-$C_6$ alkylidene group;
$R_8$ is selected from the group consisting of -H, $C_1$-$C_6$ alkyl, $C_2$-$C_6$ alkenyl, unsubstituted $C_6$-$C_{14}$ aryl, substituted $C_6$-$C_{14}$ aryl and benzyl.

A second aspect of the invention relates to a method for the synthesis of the compounds of the invention, wherein a compound of formula (II), or stereoisomers, salts or solvates thereof, reacts with a compound of formula (III), or stereoisomers, salts or solvates thereof

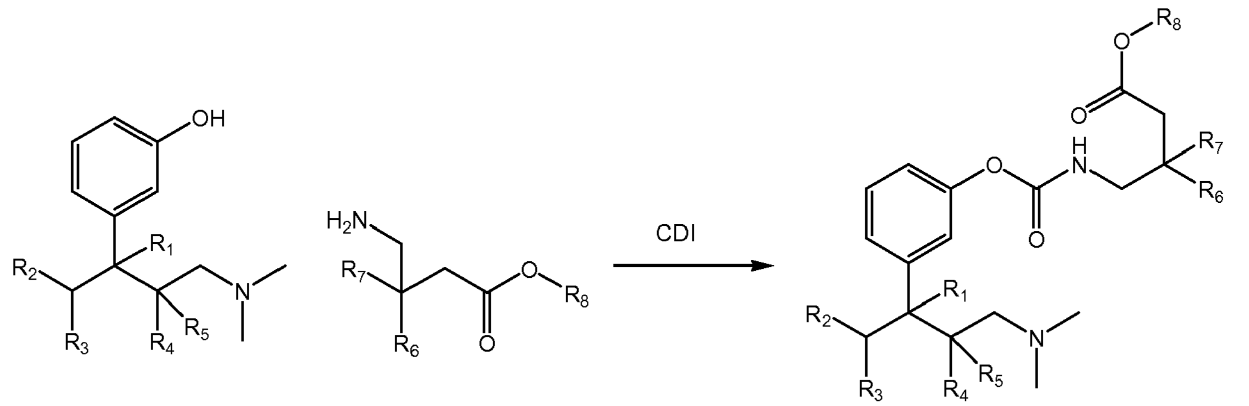


(II) (III) (I)

wherein $R_1$ to $R_8$ are as defined above.

A further aspect is a compound of the invention for use as a medicament.

A further aspect is a compound of the invention for use in the treatment of pain.

A further aspect is the use of a compound of the invention for the preparation of a medicament for the treatment of pain.

A further aspect is a method of treating pain, which method comprises administering to a patient in need of such a treatment a therapeutically effective amount of at least one compound of formula (I) or a pharmaceutical composition thereof.

A further aspect is directed to a pharmaceutical composition comprising a compound of the invention and a pharmaceutically acceptable carrier.

## DETAILED DESCRIPTION OF THE INVENTION

### Compounds of formula (I)

The invention also provides salts of compounds of the invention. For instance, pharmaceutically acceptable salts of compounds provided herein may be acid addition salts, base addition salts or metallic salts, and they can be synthesized from the parent compound which contains a basic or an acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, bisulphate, nitrate, phosphate, diacid phosphate and organic acid addition salts such as, for example, acetate, maleate, fumarate, citrate, lactate, salicilate, oxalate, succinate, tartrate, malate, mandelate, methanesulphonate (also referred to as mesilate) and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

It will be readily apparent to those skilled in the art that the scope of the present invention also encompasses salts which are not pharmaceutically acceptable as possible means for obtaining pharmaceutically acceptable salts.

The term "solvate" according to this invention is to be understood as meaning any form of the active compound according to the invention which has another molecule (most likely a polar solvent) attached to it via non-covalent bonding. Examples of solvates include hydrates and alcoholates, preferably $C_1$-$C_6$ alcoholates, e.g. methanolate.

It will be immediately apparent to the skilled person that the present invention encompasses all possible stereoisomers of the compounds described herein. An stereoisomer is understood by the skilled person as compounds made up of the same atoms bonded by the same sequence of bonds but having different three-dimensional structures which are not interchangeable, e.g. diastereoisomers or enantiomers. Separation methods of the different diastereoisomers or enantiomers by chemical and/or physical means are readily available to the skilled person. For example, an enantiomerically enriched mixture of enantiomers may be obtained at any stage of the synthesis by purification under chiral conditions, such as chiral supercritical fluid chromatography. Following the same methods pure enantiomers may also be isolated.

According to an embodiment of the invention, $R_6$ is hydrogen and $R_7$ is a $C_1$-$C_6$ alkyl group. According to a further embodiment of the invention, $R_6$ is hydrogen and $R_7$ is a branched $C_1$-$C_6$ alkyl group. According to a further embodiment of the invention, $R_6$ is hydrogen and $R_7$ is a $C_2$, branched $C_3$, or branched $C_4$ alkyl group, preferably an isobutyl group.

According to a preferred embodiment of the invention, $R_6$ and $R_7$ together form a $C_4$-$C_6$ alkylidene group, i.e. they form a $C_5$-$C_7$ cycloalkyl group together with the carbon atom to which they are attached. According to a further embodiment of the invention, $R_6$ and $R_7$ together form a $C_5$ alkylidene group, i.e. they form a cyclohexyl group together with the carbon atom to which they are attached.

According to an embodiment of the invention $R_8$ is selected from the group consisting of -H, $C_1$-$C_3$ alkyl group, allyl and benzyl, preferably selected from the group consisting of hydrogen atom and $C_1$-$C_2$ alkyl, more preferably a methyl group.

In a further embodiment, $R_2$ is hydrogen. In a further embodiment $R_3$ is a $C_1$-$C_3$ alkyl group, e.g. methyl. In a further embodiment, $R_1$ is hydroxyl. In a further embodiment $R_3$ and $R_4$ together form a $C_2$, $C_3$ or $C_4$ alkylidene group. In yet a further embodiment, $R_3$ and $R_4$ form a cyclohexyl or cyclohexenyl group together with the carbon atoms to which they are attached.

A further embodiment of the invention is a compound of formula (IA), or pharmaceutically acceptable, stereoisomers, salts or solvates thereof

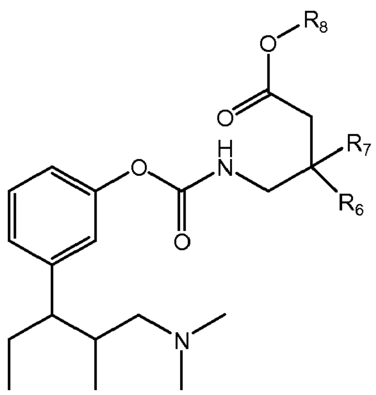

(IA)

wherein $R_6$, $R_7$ and $R_8$ are as defined above.

A further embodiment of the invention is a compound of formula (IB), or pharmaceutically acceptable, stereoisomers, salts or solvates thereof

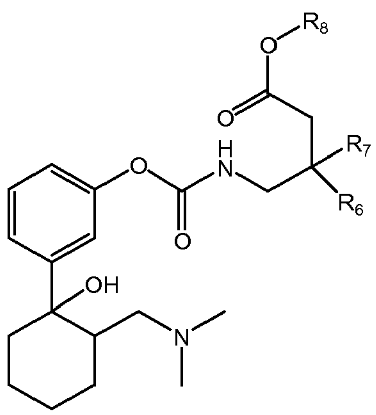

(IB)

wherein $R_6$, $R_7$ and $R_8$ are as defined above.

A further embodiment of the invention is a compound of formula (IC), or pharmaceutically acceptable, stereoisomers, salts or solvates thereof

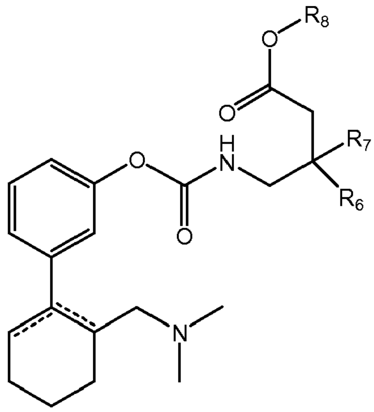

(IC)

wherein $R_6$, $R_7$ and $R_8$ are as defined above, and one of the dotted lines may represent a double bond. Thus, compounds of formula (IC) encompass compounds of formula (ICa) and compounds of formula (ICb) as shown below:

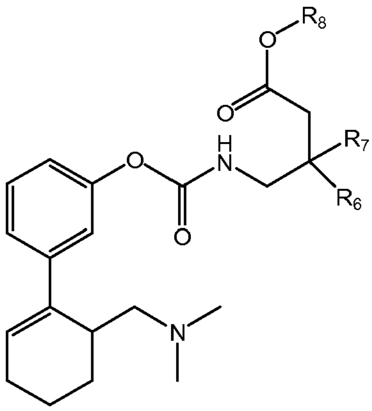

(ICa)

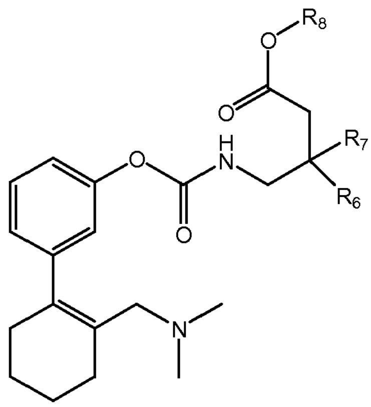

(ICb)

wherein $R_6$, $R_7$ and $R_8$ are as defined above, and one of the dotted lines may represent a double bond.

A further embodiment of the invention is a compound of formula (IE)

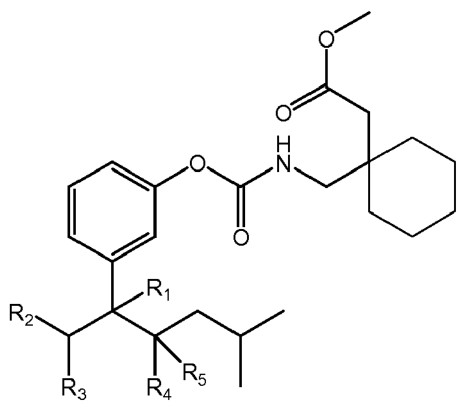

(IE)

wherein
$R_1$ is selected from the group consisting of -H, and -OH;
$R_2$ is -H;
$R_5$ is -H;
or $R_1$ and $R_2$ together form a double bond and $R_5$ is -H
or $R_1$ and $R_5$ together form a double bond and $R_2$ is -H;
$R_3$ is a $C_1$-$C_6$ alkyl group;
$R_4$ is a $C_1$-$C_6$ alkyl group;
or $R_3$ and $R_4$ together form a $C_2$-$C_4$ alkylidene group.

In a further embodiment in the compounds of formula (IE) $R_1$ is hydroxyl, $R_2$ and $R_5$ are hydrogen atoms and $R_3$ and $R_4$ together form a butylidene $-(CH_2)_4-$ group, preferably a compound having anyone of the two following formulae:

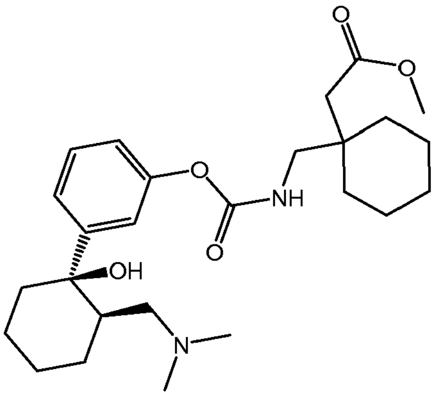

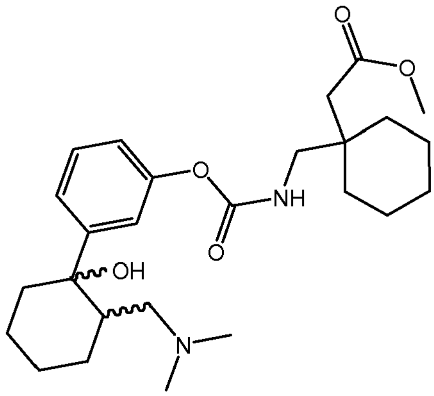

In still a further embodiment in the compounds of formula (IE) $R_1$, $R_2$ and $R_5$ are hydrogen atoms and $R_3$ and $R_4$ are methyl groups, preferably a compound having the following formula:

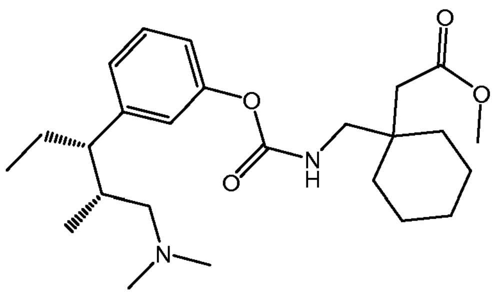

According to an embodiment, the compounds of the invention are selected from the group consisting of:

- methyl 2-(1-(((3-((2R,3S)-1-(dimethylamino)-2-methylpentan-3-yl) phenoxy) carbonylamino)methyl)cyclohexyl) acetate
- methyl 2-(1-(((3-(2-((dimethylamino)methyl)-1-hydroxycyclohexyl) phenoxy)carbonylamino)methyl)cyclohexyl) acetate
- methyl 2-(1-(((3-((1R,2R)-(2-((dimethylamino)methyl)-1-hydroxy cyclohexyl)phenoxy)carbonylamino)methyl) cyclohexyl)acetate
- methyl 2-(1-(((3-(2-((dimethylamino)methyl)cyclohex-1-enyl)phenoxy) carbonylamino)methyl)cyclohexyl)acetate
- 2-(1-(((3-((2R,3S)-1-(dimethylamino)-2-methylpentan-3-yl)phenoxy) carbonylamino)methyl)cyclohexyl)acetic acid
- 2-(1-(((3-(2-((dimethylamino)methyl)-1-hydroxycyclohexyl)phenoxy) carbonylamino)methyl)cyclohexyl)acetic acid
- ethyl 2-(1-(((3-((2R,3S)-1-(dimethylamino)-2-methylpentan-3-yl) phenoxy)carbonylamino)methyl)cyclohexyl) acetate
- ethyl 2-(1-(((3-(2-((dimethylamino)methyl)-1-hydroxycyclohexyl) phenoxy)carbonylamino)methyl)cyclohexyl) acetate
- ethyl 2-(1-(((3-((1R,2R)-2-((dimethylamino)methyl)-1-hydroxy cyclohexyl)phenoxy)carbonylamino)methyl) cyclohexyl)acetate
- ethyl 2-(1(((3-(2-((dimethylamino)methyl)cyclohex-1-enyl)phenoxy) carbonylamino)methyl)cyclohexyl)acetate

- allyl 2-(1-(((3-((2R,3S)-1-(dimethylamino)-2-methylpentan-3-yl) phenoxy)carbonylamino)methyl)cyclohexyl)acetate
- allyl 2-(1-(((3-(2-((dimethylamino)methyl)-1-hydroxycyclohexyl) phenoxy)carbonylamino)methyl)cyclohexyl)acetate
- allyl 2-(1-(((3-((1R,2R)-2-((dimethylamino)methyl)-1-hydroxy cyclohexyl)phenoxy)carbonylamino)methyl) cyclohexyl)acetate
- allyl 2-(1-(((3-(2-((dimethylamino)methyl)cyclohex-1-enyl)phenoxy) carbonylamino)methyl)cyclohexyl)acetate
- allyl 2-(1-(((3-(6-((dimethylamino)methyl)cyclohex-1-enyl)phenoxy) carbonylamino)methyl)cyclohexyl)acetate
- isopropyl 2-(1-(((3-((2R,3S)-1-(dimethylamino)-2-methylpentan-3-yl)phenoxy)carbonylamino)methyl)cyclohexyl) acetate
- isopropyl 2-(1-(((3-(2-((dimethylamino)methyl)-1-hydroxycyclohexyl) phenoxy)carbonylamino)methyl)cyclohexyl) acetate
- isopropyl 2-(1-(((3-((1R,2R)-2-((dimethylamino)methyl)-1-hydroxy cyclohexyl)phenoxy)carbonylamino)methyl) cyclohexyl)acetate
- isopropyl 2-(1-(((3-(6-((dimethylamino)methyl)cyclohex-1-enyl) phenoxy)carbonylamino)methyl)cyclohexyl)acetate
- benzyl 2-(1-(((3-((2R,3S)-1-(dimethylamino)-2-methylpentan-3-yl) phenoxy)carbonylamino)methyl)cyclohexyl) acetate
- benzyl 2-(1-(((3-(2-((dimethylamino)methyl)-1-hydroxycyclohexyl) phenoxy)carbonylamino)methyl)cyclohexyl) acetate
- benzyl 2-(1-(((3-((1R,2R)-2-((dimethylamino)methyl)-1-hydroxy cyclohexyl)phenoxy)carbonylamino)methyl) cyclohexyl)acetate
- benzyl 2-(1-(((3-(6-((dimethylamino)methyl)cyclohex-1-enyl)phenoxy) carbonylamino)methyl)cyclohexyl)acetate
- phenyl 2-(1-(((3-(2-((dimethylamino)methyl)-1-hydroxycyclohexyl) phenoxy)carbonylamino)methyl)cyclohexyl) acetate
- (R)-methyl 3-(((3-((2R,3S)-1-(dimethylamino)-2-methylpentan-3-yl) phenoxy)carbonylamino)methyl)-5-methylhexanoate
- (R)-methyl 3-(((3-((1R,2R)-2-((dimethylamino)methyl)-1-hydroxy cyclohexyl)phenoxy)carbonylamino)methyl)-5-methylhexanoate
- (R)-methyl 3-(((3-((S)-6-((dimethylamino)methyl)cyclohex-1-enyl) phenoxy)carbonylamino)methyl)-5-methylhexanoate
- (R)-allyl 3-(((3-((2R,3S)-1-(dimethylamino)-2-methylpentan-3-yl) phenoxy)carbonylamino)methyl)-5-methylhexanoate
- (R)-allyl 3-(((3-((1R,2R)-2-((dimethylamino)methyl)-1-hydroxy cyclohexyl)phenoxy)carbonylamino)methyl)-5-methylhexanoate
- (3R)-allyl 3-(((3-(6-((dimethylamino)methyl)cyclohex-1-enyl)phenoxy) carbonylamino)methyl)-5-methylhexanoate

or pharmaceutically acceptable, salts or solvates thereof.

Synthesis of the compounds of formula (I) and intermediates of the same

As mentioned above, the compounds of the invention may be prepared by reacting a compound of formula (II), or stereoisomers, salts or solvates thereof, with a compound of formula (III), or stereoisomers, salts or solvates thereof in the presence of carbonyldiimidazole (CDI).

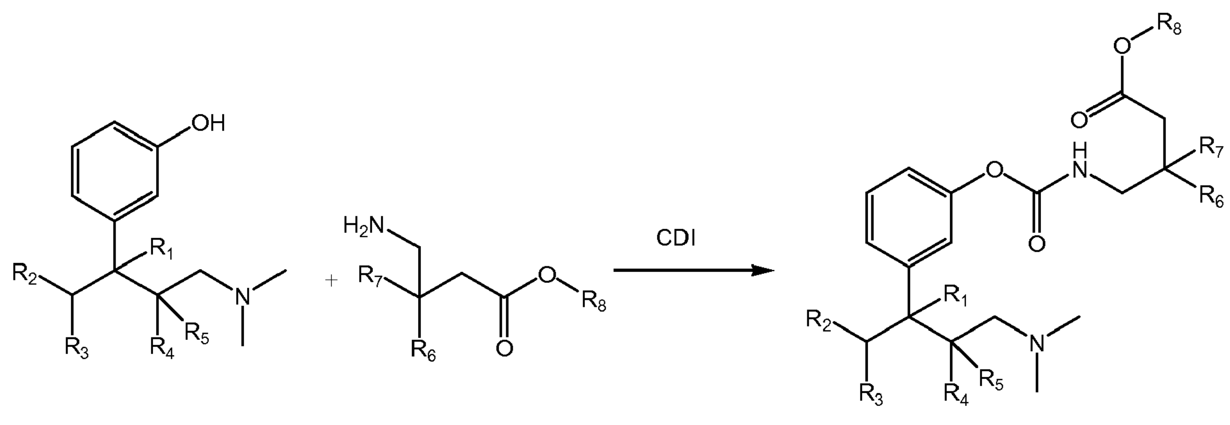


(II) (III) (I)

In the former reaction the compounds of formula (III) may be used as such or in the form of their hydrochloride salts of formula (IIIa):

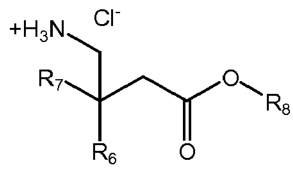

(IIIa)

Compounds of formula (III) wherein $R_8$ is different from hydrogen are conveniently prepared in the form of their hydrochloride salts of formula (IIIa) as is described below. In this case it is convenient to use the hydrochloride for the preparation of compounds of formula (I).

The reaction is carried out in inert aprotic solvents, such as halogenated solvents, ether solvents, dimethylformamide, dimethylsulfoxide and acetonitrile, preferably in dichloromethane or tetrahydrofurane at a temperature comprised in the range of -20° and 120°C (with the proviso that the solvent must be in liquid form at the selected temperature), preferably between 0° and 35°C. In an embodiment of the present invention the reaction may be carried out in the presence of an amine having a pKa comprised between 9 and12) such as triethylamine (pKa=10.78) or diisopropylethylamine (pKa=11).

Compounds of formula (II) may be prepared following the methods described in the literature (NL 6610022; Flick et al. Arzneim. Forsch/Drug Res. (1978), 28 (I), 107-113; Graham R. et al. Org. Proc. Res. Dev., 2002, 6(5), 729-737; ES 2138271; US 3564100; EP0786450; EP693475; WO2008012047).

Compounds of formula (III) may be prepared by a general esterification process wherein a compound of formula (IV) is reacted with an alcohol of formula (V) in conditions described as appropriate in the literature.

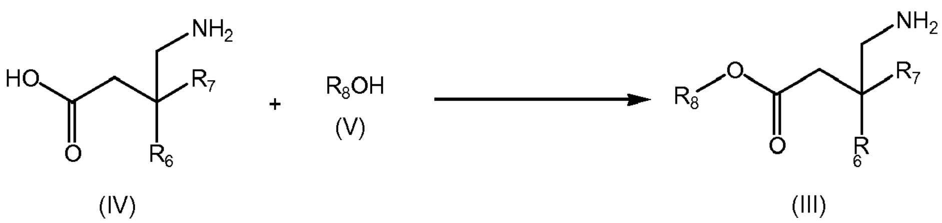

wherein $R_6$, $R_7$ and $R_8$ are as hereinabove defined.

Known conditions for these estererification reactions can be used, and appropriate conditions can be found in reference books such as chapter 10 of "Advanced Organic Chemistry: reactions, mechanisms and structures", March, J., fifth edition, Wiley-interscience (e.g. pages 484-488). Such references include preferred reaction conditions, solvents and leaving groups. Examples of appropriate conditions are:

a) reacting the compound of formula (IV) with the alcohol of formula (V) in the presence of an halogenating agent such as acetyl chloride, oxalyl chloride, tionyl chloride,...
b) reacting the compound of formula (IV) with the alcohol of formula (V) in the presence of hydrogen chloride; or
c) reacting the compound of formula (IV) with the alcohol of formula (V) in the presence of a condensation agent such as N-(3-dimethylaminopropyl)-N'-ethylcarbodiimide, dicyclohexylcarbodiimide, etc.

When compounds of formula (III) are obtained in the conditions a) or b) mentioned above they are obtained in the form of the halohydrates (IIIa) as is shown in the scheme below:

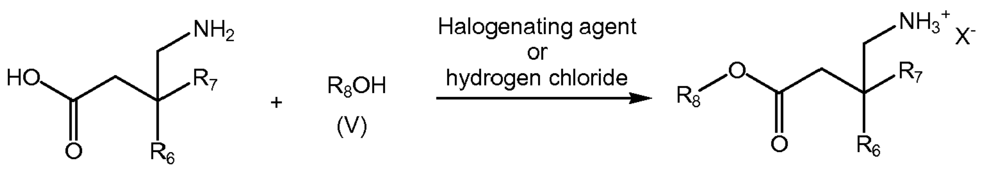


(IV) (V) (IIIa)

As explained above compounds of formula (IIIa) can be directly reacted with compounds of formula (II) to yield compounds of formula (I). Alternatively they may also be converted into the free base of formula (III) prior to reaction with compounds of formula (II).

Compounds of formula (IV) are readily available or can be synthesized by methods described in the literature, for example in US 2009/0005352 A1, WO 2008/060572 or US 4,024,175.

Compounds of formula (I) may also be prepared using an esterification process similar to the one described for the preparation of compounds of formula (III) wherein a compound of formula (ID)

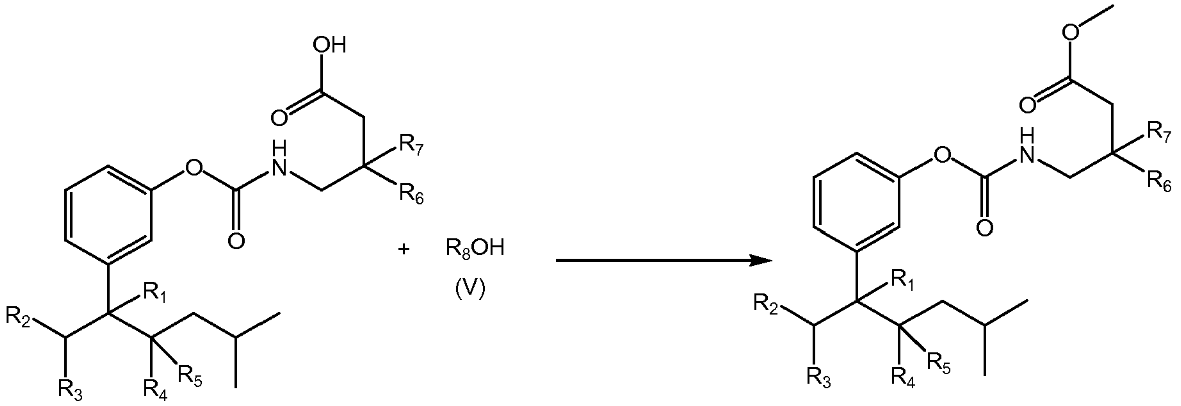

(ID) (V) (I)

is reacted with an alcohol of formula (V) in conditions described as appropriate in the literature. Known conditions for these estererification reactions can be used, and appropriate conditions can be found in reference books such as chapter 10 of "Advanced Organic Chemistry: reactions, mechanisms and structures", March, J., fifth edition, Wiley-interscience (e.g. pages 484-488). Such references include preferred reaction conditions, solvents and leaving groups. Examples of appropriate conditions are:

a) reacting the compound of formula (ID) with the alcohol of formula (V) in the presence of an halogenating agent such as acetyl chloride, oxalyl chloride, tionyl chloride,...
b) reacting the compound of formula (ID) with the alcohol of formula (V) in the presence of hydrogen chloride; and
c) reacting the compound of formula (ID) with the alcohol of formula (V) in the presence of a condensation agent such as N-(3-dimethylaminopropyl)-N'-ethylcarbodiimide, dicyclohexylcarbodiimide, etc.

In the cases a) and b) the esterification reaction yields the halohydrate of the compounds of formula (I) from which the free base products of formula (I) may be recovered.

Uses of the compounds of the invention

The term "treatment" in the context of this specification means administration of a compound or formulation of the invention to eliminate or ameliorate pain, or symptoms associated to pain.

Pain can be classified according to its symptoms, and different types of pain are defined in the IASP. In an embodiment of invention, pain is selected from the group consisting of allodynia, causalgia, hyperalgesia, hyperesthesia, hyperpathia, neuralgia, neuritis and neuropathy.

According to the IASP (IASP, Classification of chronic pain, 2nd Edition, IASP Press (2002), 210-212):

- "allodynia" is defined as "a pain due to a stimulus which does not normally provoke pain", .
- "causalgia" is defined as "a syndrome of sustained burning pain, allodynia and hyperpathia after a traumatic nerve lesion, often combined with vasomotor and sudomotor dysfunction and later trophic changes"
- "hyperalgesia" is defined as "an increased response to a stimulus which is normally painful
- "hyperesthesia" is defined as "increased sensitivity to stimulation, excluding the senses"
- "neuralgia" is defined as "Pain in the distribution of a nerve or nerves"
- "neuritis" is defined as "Inflammation of a nerve or nerves"
- "neuropathy" is defined as "a disturbance of function or pathological change in a nerve: in one nerve mononeuropathy, in several nerves mononeuropthy multiplex, if diffuse and bilateral, polyneuropathy".

For the purposes of the present invention, "chronic pain" is considered any pain that persists longer than the reasonable expected healing time for the involved tissues. In an embodiment of the invention it is considered ongoing pain lasting longer than 1 month, preferably longer than 2 months. In a further embodiment of the invention, "chronic pain" is considered any pain that persists longer than 3 months, preferably longer than 6 months.
According to an embodiment of the invention, the compounds of the invention are for use to eliminate the pain in patients with neuropathic pain, preferably patients who are refractory to traditional therapies.
There is no limitation to the condition causing the pain. In an embodiment the compounds of the invention are for use in the treatment of pain associated with, e.g., fibromyalgia syndrome, diabetic neuropathy, multiple sclerosis and/or cancer.
According to a further aspect, the present invention is directed to a pharmaceutical composition comprising a compound of the invention and a pharmaceutically acceptable carrier.
The term "carrier" refers to a diluent, adjuvant, excipient, or vehicle with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions, also buffers, isotonic agents or agents capable increasing solubility. Suitable pharmaceutical carriers are described in "Remington's Pharmaceutical Sciences" by E.W. Martin or "Tratado de Farmacia Galénica", C. Faulí i Trillo, Luzán 5, S.A. de Ediciones, 1993.
The pharmaceutical composition of the invention may be administered in the form of different preparations. Non limiting examples are preparations for oral administration, e.g. tablets, capsules, syrups or suspensions; ophthalmological administration, e.g. solutions, ointments or creams; and parenteral administration, e.g. aqueous and non-aqueous sterile injection solutions or aqueous and non-aqueous sterile suspensions. Also, the pharmaceutical compositions of the invention may include topical compositions, e.g. creams, ointments or pastes, or transdermic preparations such as patches or plasters. The pharmaceutical composition of the invention may also be prepared for vaginal or for rectal administration, e.g. rectal gel or suppository.
Generally an effective administered amount of a compound used in the invention will depend on the relative efficacy of the compound chosen, the severity of the disorder being treated, or the age, weight or mode of administration. However, active compounds will typically be administered once or more times a day, for example 1, 2, 3 or 4 times daily, with typical total daily doses in the range of from 0.01 to 100 mg/kg/day.
The compounds used in the present invention may also be administered with other drugs to provide a combination therapy. The other drugs may form part of the same composition, or be provided as a separate composition for administration at the same time or at different time.

Definitions

In the definitions of the compounds described herein the following terms have the meaning indicated:

"Alkyl" refers to a straight or branched hydrocarbon chain radical consisting of carbon and hydrogen atoms, containing no unsaturation, having the number of carbon atoms indicated in each case, and which is attached to the rest of the molecule by a single bond, e. g., methyl, ethyl, n-propyl, i-propyl, n-butyl, t-butyl, n-pentyl, etc.
"Alkenyl" refers to a straight or branched hydrocarbon chain radical consisting of carbon and hydrogen atoms, containing one unsaturation, having the number of carbon atoms indicated in each case, and which is attached to the rest of the molecule by a single bond, e. g., vinyl, allyl, i-propenyl, buten-3-enyl, buten-2-enyl, buten-1-enyl, 1-methylpropenyl, 1-methylenepropyl, 1-methylallyl, etc.
"Alkylidene" is understood as an alkyl group as defined above but attached to the rest of the molecule by two single bonds.
"Aryl" refers to an aromatic hydrocarbon radical having the number of carbon atoms indicated in each case, preferably, six to ten carbon atoms, such as phenyl or naphthyl.

Unless otherwise indicated, alkyl, cycloalkyl, alkenyl, alkynyl, aryl, aralkyl and heteroaryl radicals may be optionally substituted by one, two or three substituents such as halo, alkyl, alkenyl, alkynyl, cycloalkyl, hydroxy, alkoxy, sulfoxy, OBenzyl, OBenzoyl, carboxy, cyano, carbonyl, acyl, alkoxycarbonyl, amino, imino and nitro.

## Experimental section

The examples set forth in this description detail the suitable processes for obtaining several compounds which can be assigned to formula (I). In view of said examples, it is evident and direct for a person skilled in the art to obtain the compounds which are not explicitly exemplified, by means of applying modifications of the methods set forth, characteristic of the common general knowledge of the persons skilled in the art.

Consequently, the examples set forth below must not be interpreted as limiting the scope of the present invention, but rather as an additional and more detailed explanation which guides the person skilled in the art to a deeper understanding of the invention.

The following abbreviations are used in the following examples:

| | |
|---|---|
| AcOEt | Ethyl acetate |
| AcOH | Acetic acid |
| CDI | Carbonyldiimidazole |
| DMSO-$d_6$ | Hexadeuterodimethyl sulfoxide |
| DMF | Dimethylformamide |
| DMAP | 4-Dimethylaminopyridine |
| EDAC | N-(3-Dimethylaminopropyl)-N'-ethylcarbodiimide |
| EtOH | Ethanol |
| $Et_2O$ | Diethyl ether |
| HPLC | high pressure liquid chromatography |
| IPA | Isopropyl alcohol (or 2-Propanol) |
| RT | Room temperature |
| THF | Tetrahydrofuran |
| TLC | Thin layer chromatography |
| e.e. | Enantiomeric Excess |

The nuclear magnetic resonance spectra have been carried out in a Varian Gemini-200 apparatus.

The $^1H$ -NMR spectra indicate the working frequency and the solvent used to make the spectrum. The position of the signals is indicated in δ (ppm), using the signal of the protons of the solvent as reference. The reference values are 7.24 ppm for chloroform and 2.49 ppm for deuterated dimethyl sulfoxide. Within brackets are indicated the number of protons corresponding to each signal measured by electronic integration and the type of signal using the following abbreviations: s (singlet), d (doublet), dd (doublet of doublets), t (triplet), q (quadruplet), bs (broad signal). The signal assignation is indicated in some cases.

The $^{13}C$-NMR spectra indicate the working frequency and the solvent used to make the spectrum. The position of the signals is indicated in δ (ppm), using the central signal of the solvent as reference. The reference values are 77.00 ppm for chloroform and 39.50 ppm for deuterated dimethylsulfoxide.

The determination of impurities are performed by HPLC using an HPLC apparatus equipped with a photodiode array detector (scan from 200 to 450 nm, and then extract chromatogram at 201 nm). Chromatographic separation is performed using an Atlantis RPC18 column, 150 x 4.6 mm, mm, at 30 °C, with the following gradient method:

| t/min | A/% | B/% |
|---|---|---|
| 0 | 100 | 0 |
| 20 | 5 | 95 |
| 21 | 100 | 0 |
| 30 | 100 | 0 |

where A is 5 mM ammonium formate at pH 3 and B is acetonitrile. The flow rate is 1 ml·min$^{-1}$, and the compartment of the samples is set at 4 °C.

Samples are prepared at 1 mg·ml$^{-1}$: 1.5 mg of sample are weighed, then are dissolved with 1 ml of acetonitrile, and finally are added 0.5 ml of ammonium formate 5 mM at pH 3.

## A) SYNTHESIS

### Example 1. methyl 2-(1-(((3-((2R,3S)-1-(dimethylamino)-2-methylpentan-3-yl) phenoxy) carbonylamino)methyl) cyclohexyl)acetate

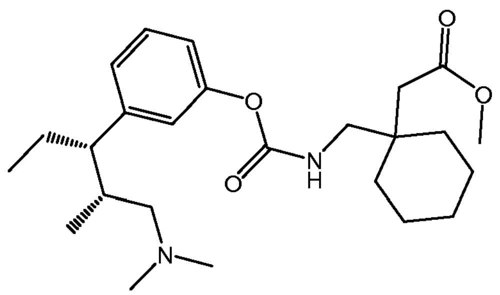

### 1A) (1-(2-methoxy-2-oxoethyl)cyclohexyl)methanaminium chloride

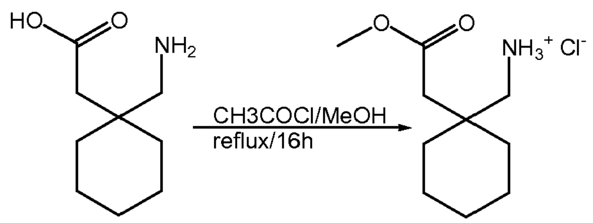


10 ml (0.14 mol) of acetyl chloride are added at 0°C to 100 ml of absolute methanol and then 8.8 g (51.6 mmol) of 2-(1-(aminomethyl)cyclohexyl)acetic are added to the methanolic solution. The mixture is allowed to reach room temperature and is subsequently heated to reach room temperature and is subsequently heated to 60°C and maintained 16h at said temperature. The mixture is concentrated to dryness and the residue is agitated in 100 ml $Et_2O$ for 15 minutes and then filtered. The solid was dried at room temperature under vacuum (< 1mm/Hg) in the presence of Sicapent® (Merck 1.00543.2800) drying agent during 24h.

8.65g (Yield 76%) of the title product were obtained.

**$^1H$ -RMN (DMSO-$d_6$, 200 MHz):** 8.15 (bs, 3H, $NH_3^+$); 3.54 (s., 3H, $OCH_3$); 2.82 (d, 2H, J=4.6 Hz, $NCH_2$); 2.47 (s, 2H, $CH_2CO_2$); 1.33 (bs, 10H)

**$^{13}C$-RMN (DMSO-$d_6$, 50 MHz):** 171.46 (1C, C=O); 51.30 (1C, $OCH_3$); 44.83 (1C, $CH_2N$); 38.22 (1C, $CH_2CO_2H$); 34.89 (1C); 32.23 (2C); 25.15 (1C); 20.65 (2C).

### 1B) methyl 2-(1-(((3-((2R,3S)-1-(dimethylamino)-2-methylpentan-3-yl)phenoxy) carbonylamino)methyl)cyclohexyl)acetate

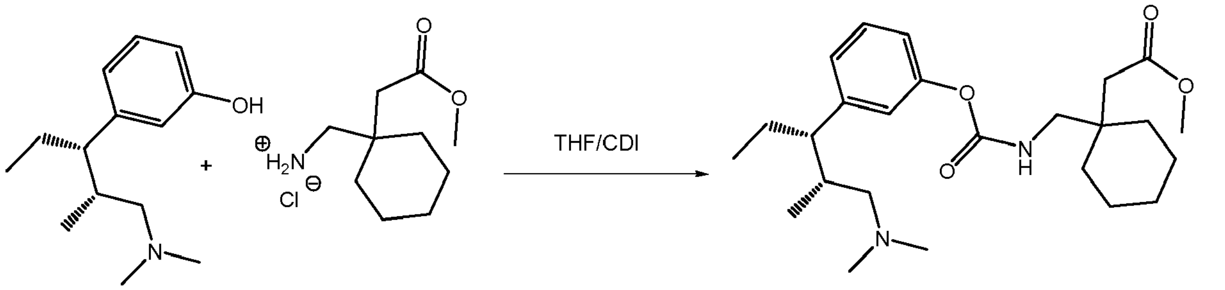


1.14g (7mmol) of CDI are added to 1.33g (6mmol) of 3-((2R, 3S)-1-(dimethylamino)-2-methylpentan-3-yl)phenol in 50 ml of THF and the mixture is heated to 50°C and maintained at said temperature for 1h. The mixture is allowed to reach room temperature and 1.33g (6mmol) of (1-(2-methoxy-2-oxoethyl)cyclohexyl)methanaminium chloride obtained in step 1A are added. The mixture is maintained at RT for 16h. The precipitated solid corresponding to the clorhydrate of the phenol starting product is filtered. The filtrate is concentrated to dryness, redissolved in 50 ml of AcOEt and washed with 2x25 ml of water. The organic phase is dried and concentrated. The residue is chromatographed on silica gel. Initial elution with 200ml of 85/5 $CH_2Cl_2$/EtOH eliminates different impurities nd further elution with 300ml of 90/10 $CH_2Cl_2$/EtOH produces 0.72g (Yield 28%) of the title product.

Purity HPLC: 96.2%

**$^1$H -RMN (DMSO-$d_6$, 200 MHz):** 7.55 (bs, 1H, NH); 7.22 (dd, 1H,); 6.92-6.80 (m., 3H); 3.50 (s., 3H, $OCH_3$); 3.11 (d, 2H, J=5.8 Hz, $NCH_2$); 2.28 (s, 2H, $CH_2CO_2$); 1.98 (s., 6H, $NCH_3$); 2.00-1.60 (m, 6H,); 1.32 (bs, 10H); 0.79 (d, J=4.8Hz, 3H, $CH_3$); 0.60 (t, J=6.6Hz, 3H, $CH_3$)

**$^{13}$C-RMN (DMSO-$d_6$, 50 MHz):** 171.75 (1C, C=O); 154.96 (1C, C=O); 151.07 (1C, C=C-O); 145.49 (1C, CH-C=CH); 128.68 (1C, phenyl); 124.80 (1C, phenyl); 121.18 (1C, phenyl); 118.96 (1C, phenyl); 63.94 (1C, $CH_2N$); 50.95 (1C, $OCH_3$); 50.17 (1C, CH-Ph); 46.95 (1C, $CH_2NH$); 45.46 (2C, $NCH_3$); 40.76 (1C); 38.89 (1C); 35.98 (1C); 32.46 (2C); 25.49 (1C); 23.09 (1C); 21.03 (2C); 15.57 (1C, $CH_3$); 12.19 (1C, $CH_3$);

### Examples 2, 3 and 4

Following a procedure analogous to the one described in example 1B), but replacing 3-((2R, 3S)-1-(dimethylamino)-2-methylpentan-3-yl)phenol by the corresponding products of formula (IIIa) the products of examples 2, 3 and 4 have been obtained:

### Example 2. methyl 2-(1-(((3-(2-((dimethylamino)methyl)-1-hydroxycyclohexyl) phenoxy)carbonylamino)methyl)cyclohexyl)acetate

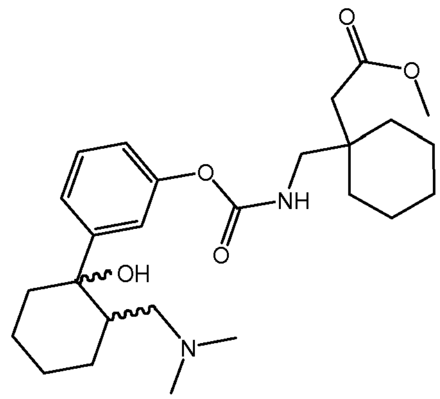

Purity HPLC: 93.9%

**$^1$H -RMN (DMSO-$d_6$, 200 MHz):** 7.55 (bs, 1H, NH); 7.21 (bs, 2H,); 7.12 (s, 1H,); 6.90-6.70 (m., 1H,); 5.10-4.80 (bs, 1H, OH); 3.53 (s., 3H, $OCH_3$); 3.13 (d, 2H, J=5.8 Hz, $NCH_2$); 2.30 (s, 2H, $CH_2CO_2$); 2.20-1.90 (m, 1H,); 1.87 (s, 6H, $NCH_3$); 2.00-1.20 (m, 20H).

**$^{13}$C-RMN (DMSO-$d_6$, 50 MHz):** 171.74 (1C, C=O); 155.06 (1C, C=O); 151.42 (1C, C=C-O); 150.87 (1C, COH-C=CH); 128.29 (1C, phenyl); 121.33 (1C, phenyl); 118.95 (1C, phenyl); 118.30 (1C, phenyl); 74.42 (1C, C-OH); 60.33 (1C, $CH_2N$); 50.97 (1C, $OCH_3$); 46.94 (1C, $CH_2NH$); 45.85 (2C, $NCH_3$); 43.12 (1C); 40.97 (1C); 39.90 (1C); 36.89 (1C); 32.44 (2C); 26.38 (1C); 25.49 (2C); 21.72 (1C); 21.02 (2C);

**Example 3. methyl 2-(1-(((3-((1R,2R)-(2-((dimethylamino)methyl)-1-hydroxy cyclohexyl)phenoxy)carbonylamino)methyl)cyclohexyl)acetate**

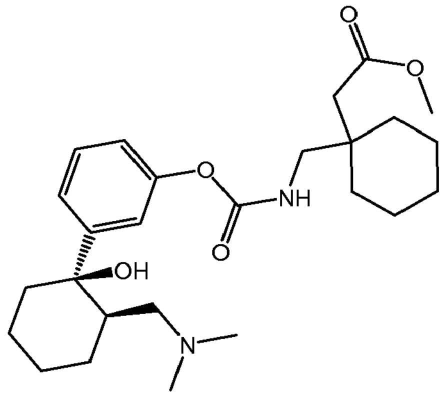

Purity HPLC: 97.2% (ee:100%)

**$^1$H -RMN (DMSO-$d_6$, 200 MHz):** 7.55 (bs, 1H, NH); 7.22 (bs, 2H,); 7.14 (s, 1H,); 6.90-6.70 (m., 1H,); 5.10-4.80 (bs, 1H, OH); 3.53 (s., 3H, $OCH_3$); 3.14 (d, 2H, J=5.8 Hz, $NCH_2$); 2.31 (s, 2H, $CH_2CO_2$); 2.20-1.90 (m, 1H,); 1.88 (s, 6H, $NCH_3$); 2.00-1.20 (m, 20H).

**$^{13}$C-RMN (DMSO-$d_6$, 50 MHz):** 171.74 (1C, C=O); 155.09 (1C, C=O); 151.43 (1C, C=C-O); 150.94 (1C, COH-C=CH); 128.30 (1C, phenyl); 121.33 (1C, phenyl); 118.97 (1C, phenyl); 118.33 (1C, phenyl); 74.49 (1C, C-OH); 60.37 (1C, $CH_2N$); 50.95 (1C, $OCH_3$); 46.97 (1C, $CH_2NH$); 45.87 (2C, $NCH_3$); 43.20 (1C); 41.02 (1C); 39.90 (1C); 36.92 (1C); 32.51 (2C); 26.44 (1C); 25.54 (2C); 21.76 (1C); 21.07 (2C);

**Example 4. methyl 2-(1-(((3-(2-((dimethylamino)methyl)cyclohex-1-enyl)phenoxy) carbonylamino)methyl)cyclohexyl)acetate**

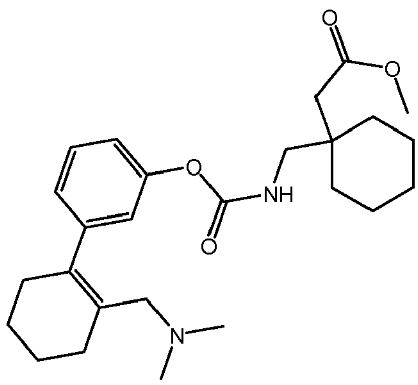

Purity HPLC: 86.9%

**$^1$H -RMN (DMSO-$d_6$, 200 MHz):** 7.60 (bs, 1H, NH); 7.35-7.18 (m., 1H,); 6.95-6.70 (m., 3H,); 3.55 (s., 3H, $OCH_3$); 3.14 (d, 2H, J=6.2 Hz, $NCH_2$); 2.69 (bs, 2H, $CH_2N$); 2.49 (m, 1H,); 2.32 (s, 2H, $CH_2CO_2$); 2.20-2.00 (m, 4H,); 1.98 (s, 6H, $NCH_3$); 1.62 (bs, 4H); 1.36 (bs, 10H); 1.37 (s, 9H, BOC)

**$^{13}$C-RMN (DMSO-$d_6$, 50 MHz):** 171.74 (1C, C=O); 154.89 (1C, C=O); 150.89 (1C, C=C-O); 144.35 (1C, COH-C=CH); 135.28 (1C, C=C); 130.68 (1C, C=C); 128.84 (1C, phenyl); 124.61 (1C, phenyl); 121.41 (1C, phenyl); 119.38 (1C, phenyl); 61.21 (1C, $CH_2N$); 50.95 (1C, $OCH_3$); 46.96 (1C, $CH_2NH$); 44.57 (2C, $NCH_3$); 40.77 (1C); 36.89 (1C);

32.46 (2C); 32.18 (1C); 27.10 (1C); 25.49 (1C); 22.80 (1C); 22.35 (1C); 21.03 (2C).

**Examples 5 and 6**

Following the procedure described for examples 1 and 2 but replacing 1-(2-methoxy-2-oxoethyl)cyclohexyl) methanaminium chloride with 2-(1-(aminomethyl) cyclohexyl)acetic the products of examples 5 and 6 have been obtained:

**Example 5. 2-(1-(((3-((2R,3S)-1-(dimethylamino)-2-methylpentan-3-yl)phenoxy) carbonylamino)methyl)cyclohexyl)acetic acid (LA13038)**

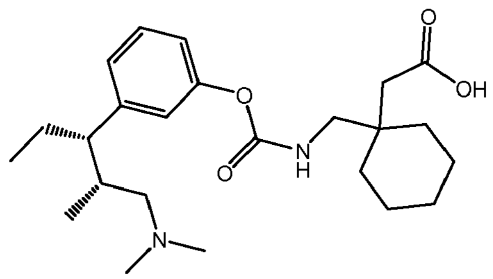

Purity HPLC: 83.3%

**$^1$H -RMN (DMSO-$d_6$, 200 MHz):** 7.80 (bs, 1H, NH); 7.22 (dd, 1H,); 6.93-6.80 (m., 2H); 6.46 (bs, 1H); 3.11 (bs, 2H, $NCH_2$); 2.30 (bs, 1H); 2.18 (s, 2H, $CH_2CO_2$); 2.02 (s., 6H, $NCH_3$); 2.00-1.40 (m, 5H,); 1.34 (bs, 10H); 0.80 (bs, 3H, $CH_3$); 0.60 (bs, 3H, $CH_3$)

**$^{13}$C-RMN (DMSO-$d_6$, 50 MHz):** 173.97 (1C, C=O); 154.87 (1C, C=O); 151.17 (1C, C=C-O); 145.40 (1C, CH-C=CH); 128.66 (1C, phenyl); 124.73 (1C, phenyl); 121.22 (1C, phenyl); 119.02 (1C, phenyl); 63.81 (1C, $CH_2N$); 50.19 (1C, CH-Ph); 47.72 (1C, $CH_2NH$); 45.30 (2C, $NCH_3$); 41.57 (1C); 36.50 (1C); 35.90 (1C); 32.81 (2C); 25.66 (1C); 23.12 (1C); 21.16 (2C); 15.59 (1C, $CH_3$); 12.20 (1C, $CH_3$);

**Example 6. 2-(1-(((3-(2-((dimethylamino)methyl)-1-hydroxycyclohexyl) phenoxy) carbonylamino)methyl)cyclohexyl)acetic acid**

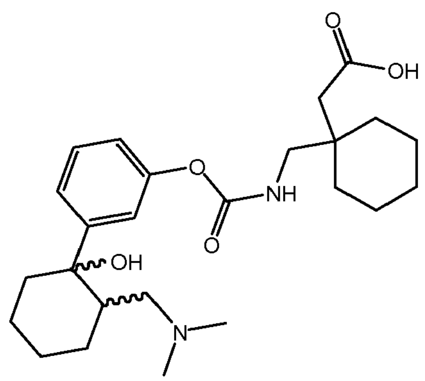

Purity HPLC: 98.1%

**$^1$H -RMN (DMSO-$d_6$, 200 MHz):** 7.80 (bs, 1H, NH); 7.23 (bs, 2H,); 7.12 (s., 1H); 6.86 (bs, 1H); 5.20-4.40 (bs, 1H, OH); 3.12 (bs, 2H, $NCH_2$); 2.30-2.10 (bs, 1H); 2.19 (s, 2H, $CH_2CO_2$); 1.95 (s., 6H, $NCH_3$); 1.90-1.20 (m, 20H,).

**$^{13}$C-RMN (DMSO-$d_6$, 50 MHz):** 173.76 (1C, C=O); 155.00 (1C, C=O); 151.16 (1C, C=<u>C</u>-O); 150.98 (1C, COH-<u>C</u>=CH); 128.39 (1C, phenyl); 121.37 (1C, phenyl); 119.14 (1C, phenyl); 118.41 (1C, phenyl); 74.35 (1C, C-OH); 60.17 (1C, $CH_2N$); 47.69 (1C, $CH_2NH$); 45.31 (2C, $NCH_3$); 42.65 (1C); 41.31 (1C); 39.90 (1C); 36.52 (1C); 32.72 (2C); 26.43 (1C); 25.64 (1C); 25.37 (1C); 21.65 (1C); 21.14 (2C);

**Example 7**

Following a procedure analogous to the one described in example 1B), but replacing 1-(2-methoxy-2-oxoethyl) cyclohexyl)methanaminium chloride (obtained in 1A) by (1-(2-ethoxy-2-oxoethyl)cyclohexyl)methanaminium chloride (obtained in 7A) the product of example 7 was obtained.

**Example 7. ethyl 2-(1-(((3-((2R,3S)-1-(dimethylamino)-2-methylpentan-3-yl) phenoxy)carbonylamino)methyl) cyclohexyl)**

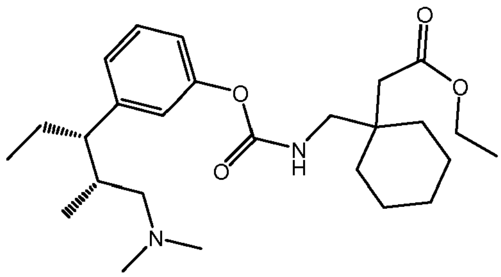

**7A) (1-(2-ethoxy-2-oxoethyl)cyclohexyl)methanaminium chloride**

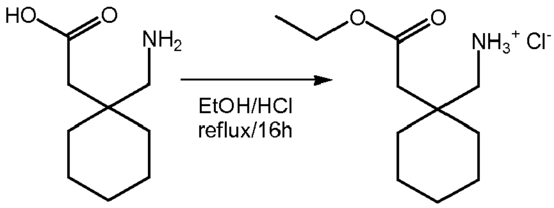


10g (58.4 mmol) of 2-(1-(aminomethyl)cyclohexyl)acetic were added to 50 ml of a 6N solution of hydrogen chloride in absolute ethanol at 0°C. The mixture is allowed to reach room temperature and is stirred at said temperature for 16h. The precipitated solid is filtered and dried. The filtrate is concentrated to a volume of 20 ml and the newly formed precipitate is filtered. Both precipitates are mixed to yield 10.65g (77%) of the title product were obtained.

**7B) ethyl 2-(1-(((3-((2R,3S)-1-(dimethylamino)-2-methylpentan-3-yl)phenoxy) carbonylamino)methyl)cyclohexyl)**

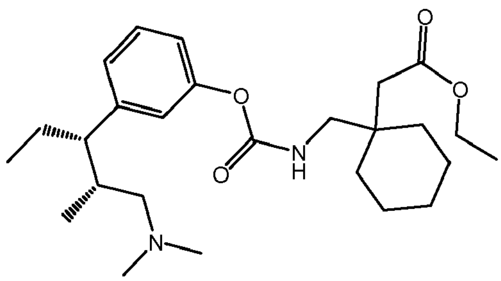

Following a procedure analogous to the one described in example 1B), but replacing 1-(2-methoxy-2-oxoethyl) cyclohexyl)methanaminium chloride (obtained in 1A) by (1-(2-ethoxy-2-oxoethyl)cyclohexyl)methanaminium chloride (obtained in 7A) the title product was obtained.

Purity HPLC: 96.5%

**$^1$H -RMN (DMSO-$d_6$, 200 MHz):** 7.60 (t., 1H, NH); 7.22 (dd, 1H,); 6.96-6.83 (m., 3H); 4.01 (q., J=7Hz, 2H, $OCH_2$); 3.50-3.30 (m, 2H); 3.15 (d, J=6.2Hz, 2H, $NCH_2$); 2.40-2.30 (m, 1H); 2.29 (s, 2H, $CH_2CO_2$); 2.01 (s., 6H, $NCH_3$); 2.00-1.60 (m, 3H,); 1.38 (bs, 10H); 0.83 (d., J=5.8Hz, 3H, $CH_3$); 0.63 (t., J=7.2Hz, 3H, $CH_3$)

**$^{13}$C-RMN (DMSO-$d_6$, 50 MHz):** 171.24 (1C, C=O); 154.91 (1C, C=O); 151.05 (1C, C=C-O); 145.49 (1C, CH-C=CH); 128.64 (1C, phenyl); 124.78 (1C, phenyl); 121.15 (1C, phenyl); 118.92 (1C, phenyl); 63.96 (1C, $CH_2N$); 59.44 (1C, $CH_2O$); 50.17 (1C, CH-Ph); 47.06 (1C, $CH_2NH$); 45.47 (2C, $NCH_3$); 40.75 (1C); 36.90 (1C); 36.00 (1C); 32.42 (2C); 25.48 (1C); 23.07 (1C); 21.01 (2C); 15.54 (1C, $CH_3$); 14.09 (1C, $CH_3$); 12.17 (1C, $CH_3$);

## Examples 8, 9 and 10

Following a procedure analogous to the one described in example 7B), but replacing 3-((2R, 3S)-1-(dimethylamino)-2-methylpentan-3-yl)phenol by the corresponding products of formula (IIIa) the products of examples 8 to 10 have been obtained:

### Example 8. ethyl 2-(1-(((3-(2-((dimethylamino)methyl)-1-hydroxy cyclohexyl) phenoxy)carbonylamino)methyl) cyclohexyl)acetate

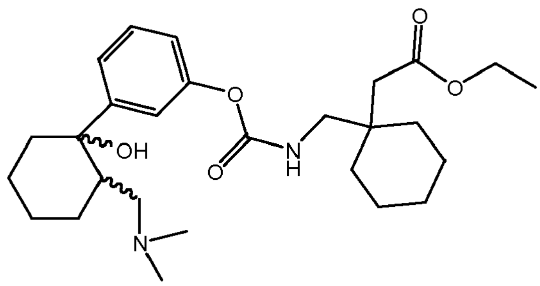

Purity HPLC: 97.3%

**$^1$H -RMN (DMSO-$d_6$, 200 MHz):** 7.60 (t., 1H, NH); 7.27 (bs, 2H,); 7.16 (s., 1H); 6.90-6.80 (m., 1H); 5.10-4.90 (bs, 1H, OH); 4.04 (q., J=7.2Hz, 2H, $OCH_2$); 3.17 (d, J=6.4Hz, 2H, $NCH_2$); 2.32 (s, 2H, $CH_2CO_2$); 2.20-2.00 (m, 1H); 1.91 (s., 6H, $NCH_3$); 1.90-1.20 (m, 20H,); 1.18 (t., J=7.2Hz, 3H, $CH_3$).

**$^{13}$C-RMN (DMSO-$d_6$, 50 MHz):** 171.12 (1C, C=O); 154.90 (1C, C=O); 151.28 (1C, C=C-O); 150.75 (1C, COH-C=CH); 128.18 (1C, phenyl); 121.21 (1C, phenyl); 118.84 (1C, phenyl); 118.18 (1C, phenyl); 74.35 (1C, C-OH); 60.29 (1C, $CH_2N$); 59.44 (1C, $CH_2O$); 47.06 (1C, $CH_2NH$); 45.82 (2C, $NCH_3$); 43.09 (1C); 40.75 (1C); 39.90 (1C); 36.91 (1C); 32.39 (2C); 26.37 (1C); 25.49 (2C); 21.72 (1C); 21.02 (2C); 14.13 (1C, $CH_3$);

### Example 9. ethyl 2-(1-(((3-((1R,2R)-2-((dimethylamino)methyl)-1-hydroxy cyclohexyl)phenoxy)carbonylamino) methyl)cyclohexyl)acetateethyl

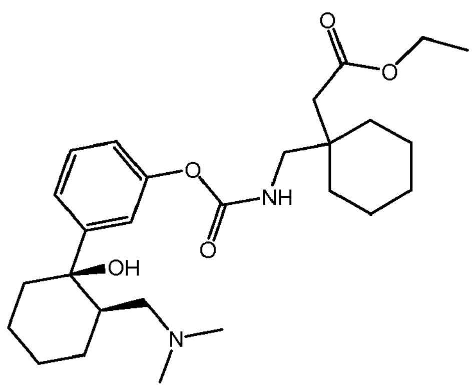

Purity HPLC: 95.9%

**$^1$H -RMN (DMSO-$d_6$, 200 MHz):** 7.56 (t., 1H, NH); 7.24 (bs, 2H,); 7.13 (s., 1H); 6.90-6.80 (m., 1H); 5.10-4.90 (bs, 1H, OH); 4.02 (q., J=7Hz, 2H, $OCH_2$); 3.15 (d, J=6.2Hz, 2H, $NCH_2$); 2.29 (s, 2H, $CH_2CO_2$); 2.20-2.00 (m, 1H); 1.88 (s., 6H, $NCH_3$); 1.90-1.20 (m, 20H,); 1.15 (t., J=7Hz, 3H, $CH_3$).

**$^{13}$C-RMN (DMSO-$d_6$, 50 MHz):** 171.25 (1C, C=O); 155.02 (1C, C=O); 151.40 (1C, C=C-O); 150.87 (1C, COH-C=CH); 128.26 (1C, phenyl); 121.21 (1C, phenyl); 118.92 (1C, phenyl); 118.27 (1C, phenyl); 74.41 (1C, C-OH); 60.32 (1C, $CH_2N$); 59.46 (1C, $CH_2O$); 47.06 (1C, $CH_2NH$); 45.84 (2C, $NCH_3$); 43.11 (1C); 40.95 (1C); 39.90 (1C); 36.91 (1C); 32.40 (2C); 26.37 (1C); 25.47 (2C); 21.71 (1C); 21.00 (2C); 14.11 (1C, $CH_3$);

**Example 10. ethyl 2-(1-(((3-(2-((dimethylamino)methyl)cyclohex-1-enyl)phenoxy) carbonylamino)methyl)cyclohexyl)acetate**

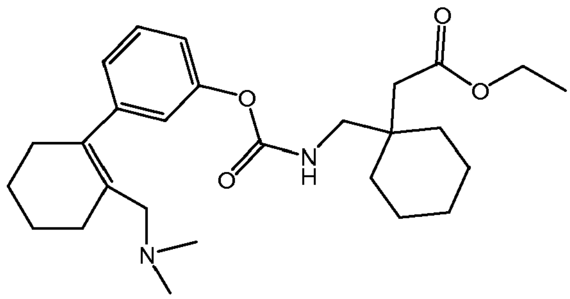

Purity HPLC: 95.4%

**$^1$H -RMN (DMSO-$d_6$, 200 MHz):** 7.40 (t., 1H, NH); 7.10-7.04 (m., 1H,); 6.73-6.57 (m., 3H); 3.80 (q., J=7Hz, 2H, $OCH_2$); 2.93 (d, J=6.2Hz, 2H, $NCH_2$); 2,28 (s, 2H, $CH_2CO_2$); 1.81 (s., 6H, $NCH_3$); 2.00-1.00 (m, 20H,); 0.94 (t., J=7Hz, 3H, $CH_3$).

**$^{13}$C-RMN (DMSO-$d_6$, 50 MHz):** 172.03 (1C, C=O); 155.62 (1C, C=O); 151.63 (1C, C=C-O); 145.10 (1C, COH-C=CH); 135.98 (1C, C=C); 131.28 (1C, C=C); 129.61 (1C, phenyl); 125.38 (1C, phenyl); 122.18 (1C, phenyl); 120.14 (1C, phenyl); 61.98 (1C, $CH_2N$); 60.24 (1C, $OCH_3$); 47.86 (1C, $CH_2NH$); 45.35 (2C, $NCH_3$); 41.54 (1C); 37.68 (1C); 33.19 (2C); 32.94 (1C); 27.84 (1C); 26.25 (1C); 23.56 (1C); 23.10 (1C); 21.78 (2C); 14.88 (1C, $CH_3$);

**Example 11. Allyl 2-(1-(((3-((2R,3S)-1-(dimethylamino)-2-methylpentan-3-yl) phenoxy)carbonylamino)methyl) cyclohexyl)acetate**

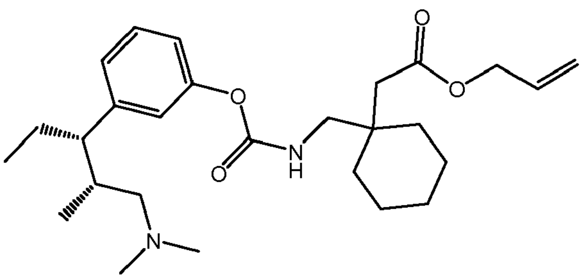

**11A) (1-(2-(allyloxy)-2-oxoethyl)cyclohexyl)methanaminium chloride**

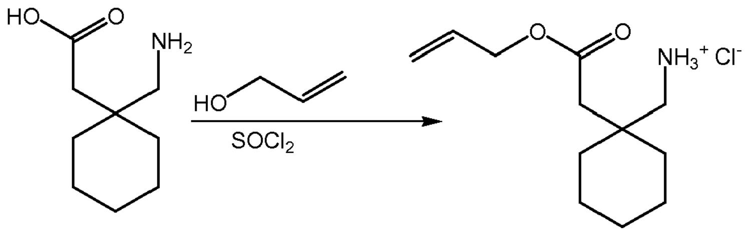


12g (70 mmol) de 2-(1-(aminomethyl) cyclohexyl)acetic were added to 70 ml allyl alcohol at 0°C in a nitrogen atmosphere. 15.63 ml of thionyl chloride are added dropwise and the mixture was allowed to reach room temperature and further stirred during 16h.

Once the reaction was finished 140 ml or $Et_2O$ were slowly added and the precipitated solid was filtered and dried. 12.16g (70%) of the title product were obtained.

**$^1H$ -RMN (DMSO-$d_6$, 200 MHz):** 8.17 (bs, 3H, $NH_3^+$); 6.00-5.80 (m, 1H, <u>CH</u>=$CH_2$); 5.24 (d.d., 2H, CH=<u>$CH_2$</u>); 4.52 (d., J=5Hz, 2H, $OCH_2$); 2.85 (d, 2H, J=4.2 Hz, $NCH_2$); 2.48 (s, 2H, $CH_2CO_2$); 1.37 (bs, 10H)

**$^{13}C$-RMN (DMSO-$d_6$, 50 MHz):** 170.59 (1C, C=O); 132.54 (1C, <u>CH</u>-$CH_2$); 117.94 (1C, CH=<u>$CH_2$</u>); 64.38 (1C, $OCH_2$); 44.80 (1C, $CH_2N$); 38.11 (1C, $CH_2CO_2H$); 34.88 (1C); 32.21 (2C); 25.07 (1C); 20.56 (2C).

**11B) Allyl 2-(1-(((3-((2R,3S)-1-(dimethylamino)-2-methylpentan-3-yl) phenoxy) carbonylamino)methyl)cyclohexyl)acetate**

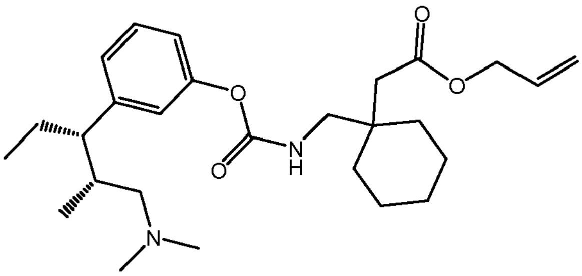

Following a procedure analogous to the one described in example 1B), but replacing 1-(2-methoxy-2-oxoethyl) cyclohexyl)methanaminium chloride (obtained in 1A) by (1-(2-(allyloxy)-2-oxoethyl)cyclohexyl)methanaminium chloride (obtained in 11A) the title product was obtained.

Purity HPLC: 93.9%

**$^1H$ -RMN (DMSO-$d_6$, 200 MHz):** 7.59 (t., 1H, NH); 7.25 (dd, 1H,); 6.96-6,83 (m., 3H); 6.00-5.80 (m, 1H, <u>CH</u>=$CH_2$); 5.33-5.15 (m, 2H, CH=<u>$CH_2$</u>); 4.50 (d., J=5.6Hz, 2H, $OCH_2$); 3.50-3.30 (m, 2H); 3.16 (d, J=6.2Hz, 2H, $NCH_2$); 2.35 (s, 2H, $CH_2CO_2$); 2.02 (s., 6H, $\overline{N}CH_3$); 2.08-1.60 (m, 4H,); 1.39 (bs, 10H); 0.83 (d., J=5.8Hz, 3H, $CH_3$); 0.63 (t., J=7Hz, 3H, $CH_3$)

**$^{13}C$-RMN (DMSO-$d_6$, 50 MHz):** 171.72 (1C, C=O); 155.72 (1C, C=O); 151.83 (1C, C=<u>C</u>-O); 146.26 (1C, CH-<u>C</u>=CH); 133.55 (1C, <u>CH</u>=$CH_2$); 129.43 (1C, phenyl); 125.57 (1C, phenyl); 121.93 (1C, CH=<u>$CH_2$</u>); 119.72 (1C, phenyl); 118.47 (1C, phenyl); 64.90 (1C, $CH_2N$); 64.72 (1C, $CH_2O$); 50.95 (1C, CH-Ph); 47.78 (1C, $CH_2NH$); 46.23 (2C, $NCH_3$); 41.12 (1C); 37.69 (1C); 36.74 (1C); 33.18 (2C); 26.24 (1C); 23.86 (1C); 21.78 (2C); 16.34 (1C, $CH_3$); 12.95 (1C, $CH_3$).

**Examples 12, 13, 14 and 15**

Following a procedure analogous to the one described in example 11B), but replacing 3-((2R, 3S)-1-(dimethylamino)-2-methylpentan-3-yl)phenol by the corresponding products of formula (IIIa) the products of examples 12 to 15 have been obtained:

**Example 12. allyl 2-(1-(((3-(2-((dimethylamino)methyl)-1-hydroxycyclohexyl) phenoxy)carbonylamino)methyl) cyclohexyl)acetate**

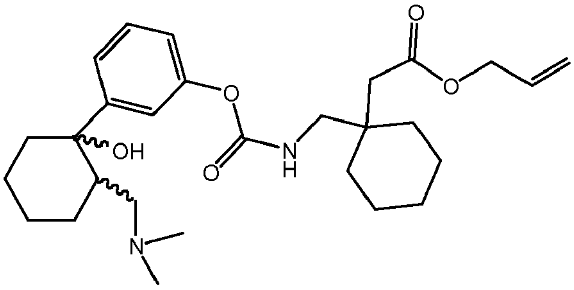

Purity HPLC: 93.2%

**$^1$H -RMN (DMSO-$d_6$, 200 MHz):** 7.60 (t., 1H, NH); 7.27 (bs, 2H,); 7.13 (s., 1H); 6.90-6.80 (m., 1H); 6.00-5.80 (m, 1H, $CH=CH_2$); 5.33-5.16 (m, 2H, $CH=CH_2$); 5.10-4.90 (bs, 1H, OH); 4.51 (d., J=5.4Hz, 2H, $OCH_2$); 3.16 (d, J=6Hz, 2H, $NCH_2$); 2.35 (s, 2H, $CH_2CO_2$); 2.20-2.00 (m, 1H); 1.88 (s., 6H, $NCH_3$); 1.90-1.20 (m, 20H,).

**$^{13}$C-RMN (DMSO-$d_6$, 50 MHz):** 170.96 (1C, C=O); 155.06 (1C, C=O); 151.41 (1C, C=C-O); 150.87 (1C, COH-C=CH); 132.77 (1C, $CH=CH_2$); 128.29 (1C, phenyl); 121.34 (1C, phenyl); 118.97 (1C, phenyl); 118.29 (1C, phenyl); 117.75 (1C, $CH=CH_2$); 74.41 (1C, C-OH); 64.15 (1C, $CH_2O$); 60.33 (1C, $CH_2N$); 47.06 (1C, $CH_2NH$); 45.85 (2C, $NCH_3$); 43.10 (1C); 40.95 (1C); 40.75 (1C); 39.90 (1C); 36.93 (1C); 32.39 (2C); 26.38 (1C); 25.48 (2C); 21.70 (1C); 21.00 (2C).

**Example 13. allyl 2-(1-(((3-((1R,2R)-2-((dimethylamino)methyl)-1-hydroxy cyclohexyl)phenoxy)carbonylamino) methyl)cyclohexyl)acetate**

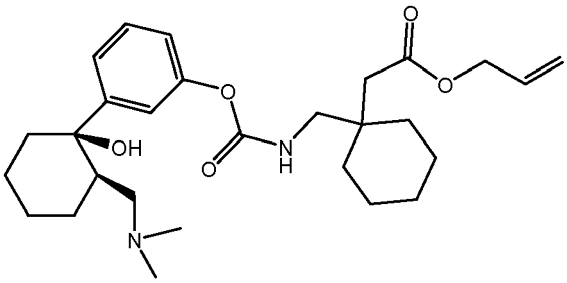

Purity HPLC: 91.1%

**$^1$H -RMN (DMSO-$d_6$, 200 MHz):** 7.62 (t., 1H, NH); 7.24 (bs, 2H,); 7.14 (s., 1H); 6.90-6.85 (m., 1H); 6.00-5.80 (m, 1H, $CH=CH_2$); 5.35-5.16 (m, 2H, $CH=CH_2$); 5.10-4.90 (bs, 1H, OH); 4.51 (d., J=5.4Hz, 2H, $OCH_2$); 3.16 (d, J=6.2Hz, 2H, $NCH_2$); 2.36 (s, 2H, $CH_2CO_2$); 2.20-2.00 (m, 1H); 1.88 (s., 6H, $NCH_3$); 1.90-1.20 (m, 20H,).

**$^{13}$C-RMN (DMSO-$d_6$, 50 MHz):** 171.74 (1C, C=O); 155.84 (1C, C=O); 152.18 (1C, C=C-O); 151.65 (1C, COH-C=CH); 133.56 (1C, $CH=CH_2$); 129.07 (1C, phenyl); 122.12 (1C, phenyl); 119.76 (1C, phenyl); 119.07 (1C, phenyl); 118.52 (1C, $CH=CH_2$); 75.18 (1C, C-OH); 64.92 (1C, $CH_2O$); 61.10 (1C, $CH_2N$); 47.77 (1C, $CH_2NH$); 46.61 (2C, $NCH_3$); 43.86 (1C); 41.74 (1C); 41.53 (1C); 39.90 (1C); 37.70 (1C); 33.16 (2C); 27.14 (1C); 26.25 (2C); 22.47 (1C); 21.78 (2C).

**Example 14. allyl 2-(1-(((3-(2-((dimethylamino)methyl)cyclohex-1-enyl)phenoxy) carbonylamino)methyl)cyclohexyl)acetate**

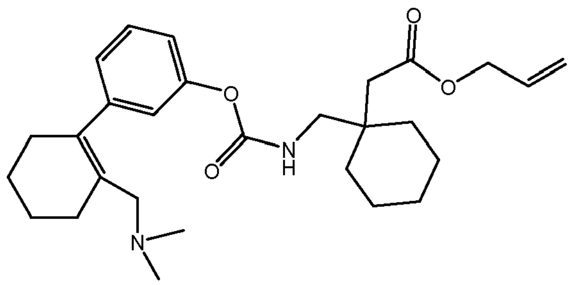

Purity HPLC: 94.5%

**$^1$H -RMN (DMSO-$d_6$, 200 MHz):** 7.64 (t., 1H, NH); 7.30-7.20 (m., 1H,); 6.98-6.72 (m., 3H); 6.00-5.80 (m, 1H, $CH=CH_2$); 5.35-5.16 (m, 2H, $CH=CH_2$); 4.51 (bs, 2H, $OCH_2$); 3.16 (bs, 2H, $NCH_2$); 2.67 (bs, 2H); 2.35 (s, 2H, $CH_2CO_2$); 2.20-2.00 (m, 4H,); 1.95 (s., 6H, $NCH_3$); 1.62 (bs, 4H,); 1.38 (bs, 10H,).

**$^{13}$C-RMN (DMSO-$d_6$, 50 MHz):** 170.94 (1C, C=O); 154.86 (1C, C=O); 150.84 (1C, C=C-O); 144.35 (1C, COH-C=CH); 135.08 (1C, C=C); 132.76 (1C, $CH=CH_2$); 130.88 (1C, C=C); 128.82 (1C, phenyl); 124.63 (1C, phenyl); 121.41 (1C, phenyl); 119.37 (1C, phenyl); 117.70 (1C, $CH=CH_2$); 64.13 (1C, $CH_2O$); 61.25 (1C, $CH_2N$); 47.00 (1C, $CH_2NH$); 44.62 (2C, $NCH_3$); 40.75 (1C); 36.90 (1C); 32.39 (2C); 32.13 (1C); 27.07 (1C); 25.46 (1C); 22.77 (1C); 22.34 (1C); 20.97 (2C).

**Example 15. allyl 2-(1-(((3-(6-((dimethylamino)methyl)cyclohex-1-enyl)phenoxy) carbonylamino)methyl)cyclohexyl)acetate**

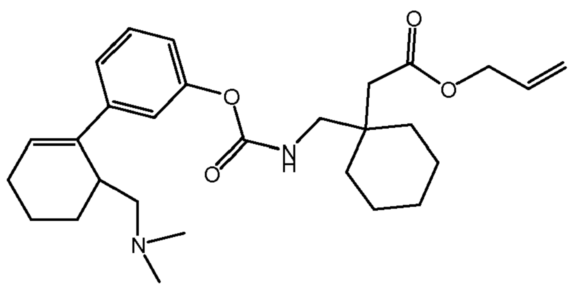

Purity HPLC: 92.3%

**$^1$H -RMN (DMSO-$d_6$, 200 MHz):** 7.63 (t., 1H, NH); 7.28 (d.d., 1H,); 7.15 (d, 1H); 7.01 (bs, 1H); 6.92 (d., 1H); 6.00-5.80 (m, 1H, $CH=CH_2$); 5.35-5.16 (m, 2H, $CH=CH_2$); 4.50 (d., J=5.6Hz, 2H, $OCH_2$); 3.15 (d, J=6.2Hz, 2H, $NCH_2$); 2.35 (s, 2H, $CH_2CO_2$); 2.05 (s., 6H, $NCH_3$); 2.20-1.50 (m, 8H,); 1.38 (bs, 10H,).

**$^{13}$C-RMN (DMSO-$d_6$, 50 MHz):** 170.93 (1C, C=O); 154.95 (1C, C=O); 151.24 (1C, C=C-O); 142.99 (1C, COH-C=CH); 138.77 (1C, C=C); 132.76 (1C, $CH=CH_2$); 128.99 (1C, C=C); 127.29 (1C, phenyl); 122.14 (1C, phenyl); 119.83 (1C, phenyl); 118.78 (1C, phenyl); 117.69 (1C, $CH=CH_2$); 64.12 (1C, $CH_2O$); 61.32 (1C, $CH_2N$); 47.00 (1C, $CH_2NH$); 45.31 (2C, $NCH_3$); 40.33 (1C); 36.92 (1C); 32.77 (1C); 32.37 (2C); 25.57 (1C); 25.46 (1C); 24.64 (1C); 20.99 (2C); 17.13 (1C).

**Example 16. Isopropyl 2-(1-(((3-((2R,3S)-1-(dimethylamino)-2-methylpentan-3-yl)phenoxy)carbonylamino)methyl)cyclohexyl)acetate**

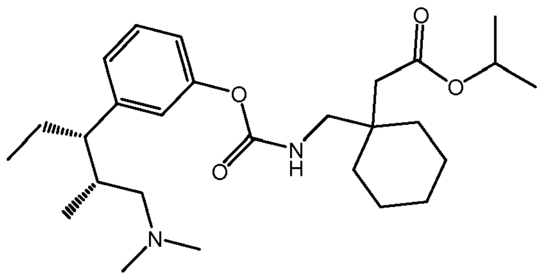

16A) (1-(2-isopropoxy-2-oxoethyl)cyclohexyl)methanaminium chloride

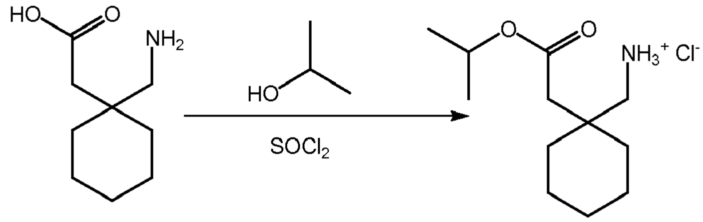


Following a procedure analogous to the one described in example 11A), but replacing allyl alcohol by 2-propyl alcohol the title product was obtained.

**$^1$H -RMN (DMSO-$d_6$, 200 MHz):** 8.13 (bs, 3H, $NH_3^+$); 5.00-4.80 (m, 1H, OCH); 2.85 (d, 2H, J=4.8Hz, $NCH_2$); 2.48 (s, 2H, $CH_2CO_2$); 1.37 (bs, 10H); 1.16 (d, J=6.2Hz, 6H, $2CH_3$)

**$^{13}$C-RMN (DMSO-$d_6$, 50 MHz):** 170.34 (1C, C=O); 67.26 (1C, OCH); 45.00 (1C, $CH_2N$); 38.51 (1C, $CH_2CO_2H$); 35.01 (1C); 32.26 (2C); 25.09 (1C); 21.59 (2C. $CH_3$); 20.59 (2C).

**16B) Isopropyl 2-(1-(((3-((2R,3S)-1-(dimethylamino)-2-methylpentan-3-yl) phenoxy)carbonylamino)methyl)cyclohexyl)acetate**

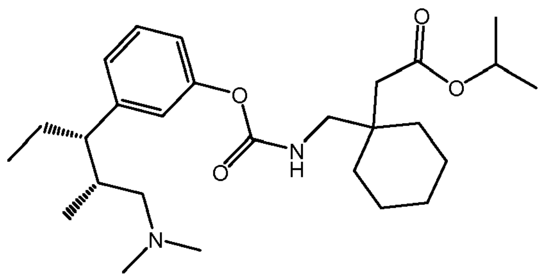

Following a procedure analogous to the one described in example 1B), but replacing 1-(2-methoxy-2-oxoethyl) cyclohexyl)methanaminium chloride (obtained in 1A) by (1-(2-isopropoxy-2-oxoethyl)cyclohexyl)methanaminium chloride (obtained in 16A) the title product was obtained.

Purity HPLC: 90.4%

**$^1$H -RMN (DMSO-$d_6$, 200 MHz):** 7.56 (t., 1H, NH); 7.25 (dd, 1H,); 6.96-6.83 (m., 3H); 4.86 (m., 1H, OCH); 3.14 (d, J=6.4Hz, 2H, $NCH_2$); 2.50-2.30 (m, 1H); 2.26 (s, 2H, $CH_2CO_2$); 2.01 (s., 6H, $NCH_3$); 2.09-1.60 (m, 5H,); 1.39 (bs, 10H); 1.14 (d, J=9.6Hz, 6H, $2CH_3$); 0.83 (d., J=6Hz, 3H, $CH_3$); 0.63 (t., J=7Hz, 3H, $CH_3$)

**$^{13}$C-RMN (DMSO-$d_6$, 50 MHz):** 170.72 (1C, C=O); 154.89 (1C, C=O); 151.04 (1C, C=<u>C</u>-O); 145.49 (1C, CH-<u>C</u>=CH); 128.65 (1C, phenyl); 124.80 (1C, phenyl); 121.16 (1C, phenyl); 118.93 (1C, phenyl); 68.47 (1C, CHO); 63.95 (1C, $CH_2N$); 50.16 (1C, CH-Ph); 47.21 (1C, $CH_2NH$); 45.48 (2C, $NCH_3$); 40.75 (1C); 36.95 (1C); 36.17 (1C); 32.38 (2C); 29.56 (1C); 25.47 (1C); 21.61 (2C, $CH_3$); 21.01 (2C); 15.57 (1C, $CH_3$); 12.17 (1C, $CH_3$);

**Examples 17, 18 and 19**

Following a procedure analogous to the one described in example 16B), but replacing 3-((2R, 3S)-1-(dimethylamino)-2-methylpentan-3-yl)phenol by the corresponding products of formula (IIIa) the products of examples 17 to 19 have been obtained: **Example 17. isopropyl 2-(1-(((3-(2-((dimethylamino)methyl)-1-hydroxycyclohexyl) phenoxy) carbonylamino)methyl)cyclohexyl)acetate**

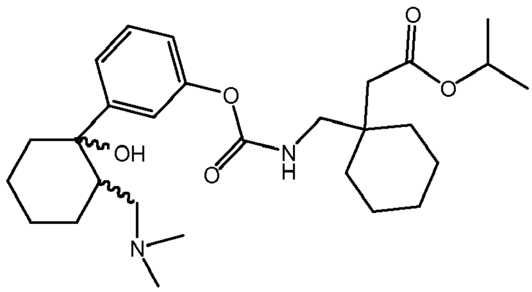

Purity HPLC: 90.5%

**$^1H$ -RMN (DMSO-$d_6$, 200 MHz):** 7.60 (t., 1H, NH); 7.24 (bs, 2H,); 7.14 (s., 1H); 6.90-6.85 (m., 1H); 4.87 (m., 1H, OCH); 4.40-4.20 (bs, 1H, OH); 3.14 (d, J=6Hz, 2H, $NCH_2$); 2.26 (s, 2H, $CH_2CO_2$); 2.20-2.00 (m, 1H); 1.88 (s., 6H, $NCH_3$); 1.90-1.20 (m, 20H,); 1.14 (d, J=6.2Hz, 6H, $2CH_3$).

**$^{13}C$-RMN (DMSO-$d_6$, 50 MHz):** 170.77 (1C, C=O); 155.01 (1C, C=O); 151.39 (1C, C=<u>C</u>-O); 150.87 (1C, COH-<u>C</u>=CH); 128.28 (1C, phenyl); 121.33 (1C, phenyl); 118.95 (1C, phenyl); 118.29 (1C, phenyl); 74.41 (1C, C-OH); 66.76 (1C, CHO); 60.32 (1C, $CH_2N$); 47.22 (1C, $CH_2NH$); 45.84 (2C, $NCH_3$); 43.11 (1C); 40.97 (1C); 40.75 (1C); 39.90 (1C); 36.98 (1C); 32.36 (2C); 26.38 (1C); 25.49 (2C); 25.35 (1C); 21.63 (2C, $CH_3$); 21.02 (2C).

**Example 18. isopropyl 2-(1-(((3-((1R,2R)-2-((dimethylamino)methyl)-1-hydroxy cyclohexyl)phenoxy)carbonylamino)methyl) cyclohexyl)acetate**

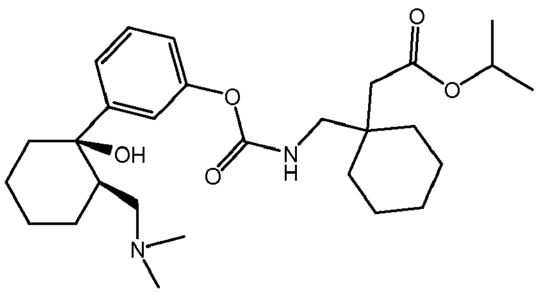

Purity HPLC: 98.4%

**$^1H$ -RMN (DMSO-$d_6$, 200 MHz):** 7.56 (t., 1H, NH); 7.24 (bs, 2H,); 7.14 (s., 1H); 6.90-6.85 (m., 1H); 4.86 (m., 1H, OCH); 4.40-4.20 (bs, 1H, OH); 3.14 (d, J=6Hz, 2H, $NCH_2$); 2.26 (s, 2H, $CH_2CO_2$); 2.20-2.00 (m, 1H); 1.90 (s., 6H, $NCH_3$); 1.90-1.20 (m, 20H,); 1.15 (d, J=6.2Hz, 6H, $2CH_3$).

**$^{13}C$-RMN (DMSO-$d_6$, 50 MHz):** 170.79 (1C, C=O); 155.03 (1C, C=O); 151.36 (1C, C=<u>C</u>-O); 150.88 (1C, COH-<u>C</u>=CH); 128.32 (1C, phenyl); 121.34 (1C, phenyl); 119.00 (1C, phenyl); 118.32 (1C, phenyl); 74.39 (1C, C-OH); 66.77 (1C, CHO); 60.29 (1C, $CH_2N$); 47.22 (1C, $CH_2NH$); 45.77 (2C, $NCH_3$); 43.02 (1C); 40.94 (1C); 40.76 (1C); 39.90 (1C); 36.98 (1C); 32.35 (2C); 26.36 (1C); 25.48 (3C); 21.64 (2C, $CH_3$); 21.02 (2C).

**Example 19. isopropyl 2-(1-(((3-(6-((dimethylamino)methyl)cyclohex-1-enyl) phenoxy)carbonylamino)methyl) cyclohexyl)acetate**

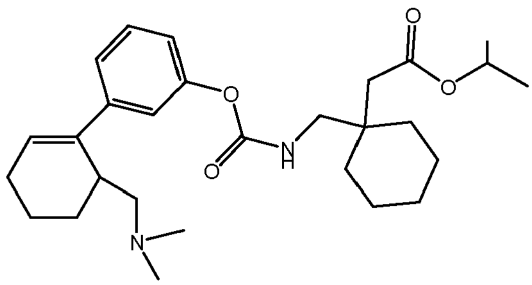

Purity HPLC: 93.2%

**$^1H$ -RMN (DMSO-$d_6$, 200 MHz):** 7.60 (t., 1H, NH); 7.28 (d.d., 1H,); 7.15 (d, 1H); 7.01 (bs, 1H); 6.92 (d., 1H); 5.98 (m, 1H, CH=C); 4.86 (m., 1H, OCH); 3.50-3.30 (m, 2H); 3.14 (d, J=6.2Hz, 2H, $NCH_2$); 2.26 (s, 2H, $CH_2CO_2$); 2.06 (s., 6H, $NCH_3$); 2.20-1.40 (m, 6H); 1.38 (bs, 10H,); 1.15 (d, J=6.2Hz, 6H, $2CH_3$).

**$^{13}C$-RMN (DMSO-$d_6$, 50 MHz):** 170.78 (1C, C=O); 154.94 (1C, C=O); 151.26 (1C, C=C-O); 142.99 (1C, phenyl); 138.77 (1C, C=C); 129.02 (1C, C=C); 127.33 (1C, phenyl); 122.15 (1C, phenyl); 119.83 (1C, phenyl); 118.82 (1C, phenyl); 66.75 (1C, CHO); 61.32 (1C, $CH_2N$); 47.22 (1C, $CH_2NH$); 45.30 (2C, $NCH_3$); 40.76 (1C); 37.00 (1C); 32.77 (1C); 32.34 (2C); 25.59 (1C); 25.47 (1C); 24.65 (1C); 21.63 (2C, $CH_3$); 21.01 (2C); 17.14(1C).

**Example 20. benzyl 2-(1-(((3-((2R,3S)-1-(dimethylamino)-2-methylpentan-3-yl) phenoxy)carbonylamino)methyl) cyclohexyl)acetate**

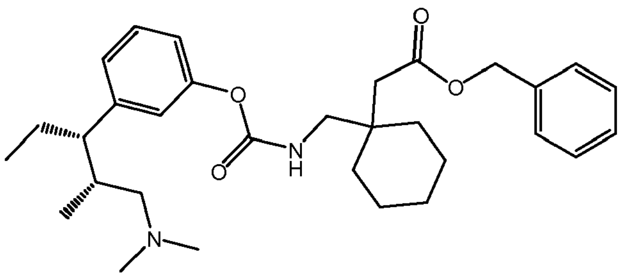

**20A) (1-(2-(benzyloxy)-2-oxoethyl)cyclohexyl)methanaminium chloride**

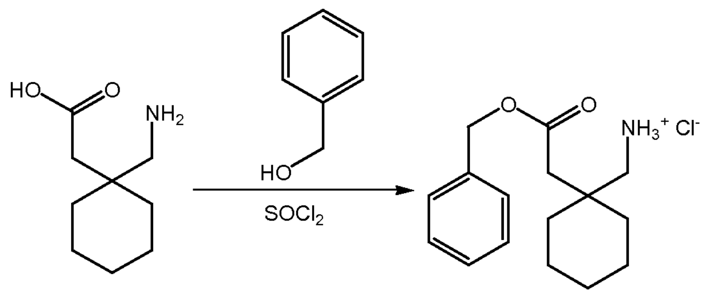


Following a procedure analogous to the one described in example 11A), but replacing allyl alcohol by benzyl alcohol the title product was obtained.

**$^1H$ -RMN (DMSO-$d_6$, 200 MHz):** 8.14 (bs, 3H, $NH_3^+$); 7.40-7.30 (m, 5H); 5.07 (s, 2H, $OCH_2$); 2.86 (bs, 2H, $NCH_2$); 2.48 (s, 2H, $CH_2CO_2$); 1.37 (bs, 1OH);

**$^{13}C$-RMN (DMSO-$d_6$, 50 MHz):** 170.87(1C, C=O); 135.99 (1C, C=CH, benzyl); 128.42 (2C, benzyl); 128.10 (2C, benzyl); 128.05 (1C, benzyl); 65.56 (1C, $OCH_2$); 44.92 (1C, $CH_2N$); 38.51 (1C, $CH_2CO_2$ 35.02 (1C); 32.22 (2C); 25.08 (1C); 20.59 (2C).

**20B) benzyl 2-(1-(((3-((2R,3S)-1-(dimethylamino)-2-methylpentan-3-yl)phenoxy) carbonylamino)methyl)cyclohexyl)acetate**

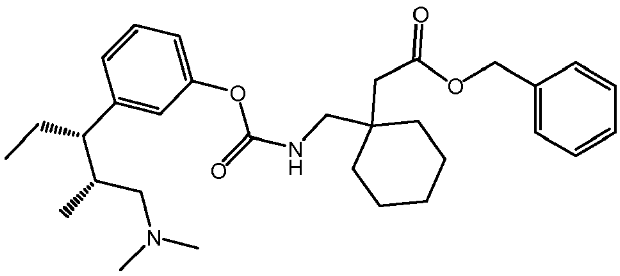

Following a procedure analogous to the one described in example 1B), but replacing 1-(2-methoxy-2-oxoethyl) cyclohexyl)methanaminium chloride (obtained in 1A) by (1-(2-(benzyloxy)-2-oxoethyl)cyclohexyl)methanaminium chloride (obtained in 20A) the title product was obtained.

Purity HPLC: 93.3%

**$^1$H -RMN (DMSO-$d_6$, 200 MHz):** 7.59 (t., 1H, NH); 7.40-7.20 (m, 5H); 7.00-6.83 (m., 4H); 5.05 (s, 2H, $OCH_2Ph$); 3.16 (d, J=6.2Hz, 2H, $NCH_2$); 2.38 (s, 2H, $CH_2CO_2$); 2.01 (s., 6H, $NCH_3$); 2.01-1.60 (m, 6H,); 1.39 (bs, 10H); 0.83 (d., J=5.6Hz, 3H, $CH_3$); 0.63 (t., J=7.2Hz, 3H, $CH_3$)

**$^{13}$C-RMN (DMSO-$d_6$, 50 MHz):** 171.16 (1C, C=O); 154.93 (1C, C=O); 151.02 (1C, C=C-O); 145.48 (1C, CH-C=CH); 136.18 (1C, C=CH, Benzyl); 128.64 (1C, phenyl); 128.51 (2C, benzyl); 128.35 (2C, benzyl); 127.98 (1C, benzyl); 124.77 (1C, phenyl); 121.15 (1C, phenyl); 118.92 (1C, phenyl); 65.25 (1C, $CH_2O$) 63.91 (1C, $CH_2N$); 50.14 (1C, CH-Ph); 46.99 (1C, $CH_2NH$); 45.44 (2C, $NCH_3$); 41.12 (1C); 37.69 (1C); 36.98 (1C); 32.37 (2C); 25.43 (1C); 23.07 (1C); 20.98 (2C); 15.55 (1C, $CH_3$); 12.17 (1C, $CH_3$).

**Examples 21, 22 and 23**

Following a procedure analogous to the one described in example 20B), but replacing 3-((2R, 3S)-1-(dimethylamino)-2-methylpentan-3-yl)phenol by the corresponding products of formula (IIIa) the products of examples 21 to 23 have been obtained:

**Example 21. benzyl 2-(1-(((3-(2-((dimethylamino)methyl)-1-hydroxy cyclohexyl)phenoxy)carbonylamino)methyl)cyclohexyl)acetate**

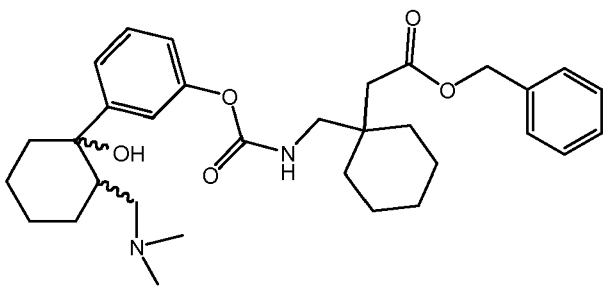

Purity HPLC: 91.5%

**$^1$H -RMN (DMSO-$d_6$, 200 MHz):** 7.61 (t., 1H, NH); 7.40-7.20 (m, 5H); 7.24 (bs, 2H,); 7.14 (s., 1H); 6.90-6.80 (m., 1H); 5.04 (s, 2H, $OCH_2$); 4.40-4.30 (bs, 1H, OH); 3.16 (d, J=6Hz, 2H, $NCH_2$); 2.38 (s, 2H, $CH_2CO_2$); 2.20-2.00 (m, 1H); 1.88 (s., 6H, $NCH_3$); 1.90-1.20 (m, 20H,).

**$^{13}$C-RMN (DMSO-$d_6$, 50 MHz):** 171.18 (1C, C=O); 155.06 (1C, C=O); 151.39 (1C, C=C-O); 150.87 (1C, COH-C=CH);); 136.18 (1C, C=CH, Benzyl); 128.37 (1C, phenyl); 128.30 (2C, benzyl); 128.03 (2C, benzyl); 127.95 (1C, benzyl); 121.34 (1C, phenyl); 118.97 (1C, phenyl); 118.29 (1C, phenyl); 74.40 (1C, C-OH); 65.29 (1C, $CH_2O$) 60.32 (1C,

$CH_2N$); 46.99 (1C, $CH_2NH$); 45.81 (2C, $NCH_3$); 43.05 (1C); 40.75 (1C); 39.90 (1C); 37.02 (1C); 32.37 (2C); 26.36 (1C); 25.47 (2C); 21.69 (1C); 21.00 (2C).

**Example 22. benzyl 2-(1-(((3-((1R,2R)-2-((dimethylamino)methyl)-1-hydroxy cyclohexyl)phenoxy)carbonylamino)methyl)cyclohexyl)acetate**

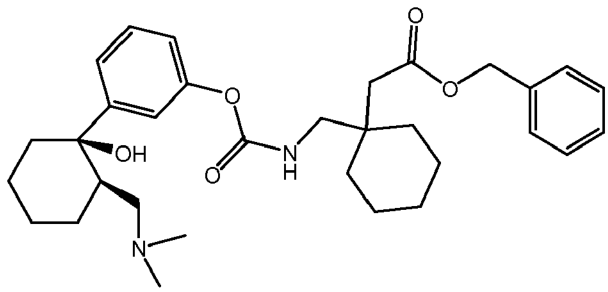

Purity HPLC: 93.4%

**$^1H$ -RMN (DMSO-$d_6$, 200 MHz):** 7.61 (t., 1H, NH); 7.40-7.20 (m, 5H); 7.24 (bs, 2H,); 7.14 (s., 1H); 6.90-6.80 (m., 1H); 5.05 (s, 2H, $OCH_2$); 4.40-4.30 (bs, 1H, OH); 3.16 (d, J=6Hz, 2H, $NCH_2$); 2.38 (s, 2H, $CH_2CO_2$); 2.20-2.00 (m, 1H); 1.88 (s., 6H, $NCH_3$); 1.90-1.20 (m, 20H,).

**$^{13}C$-RMN (DMSO-$d_6$, 50 MHz):** 171.19 (1C, C=O); 155.07 (1C, C=O); 151.40 (1C, C=C-O); 150.87 (1C, COH-C=CH); 136.21 (1C, C=CH, Benzyl); 128.38 (1C, phenyl); 128.32 (2C, benzyl); 128.04 (2C, benzyl); 127.96 (1C, benzyl); 121.34 (1C, phenyl); 118.98 (1C, phenyl); 118.29 (1C, phenyl); 74.41 (1C, C-OH); 65.29 (1C, $CH_2O$) 60.32 (1C, $CH_2N$); 47.00 (1C, $CH_2NH$); 45.82 (2C, $NCH_3$); 43.06 (1C); 40.96 (1C); 39.90 (1C); 37.02 (1C); 32.37 (2C); 26.37 (1C); 25.46 (2C); 21.70 (1C); 21.01 (2C).

**Example 23. benzyl 2-(1-(((3-(6-((dimethylamino)methyl)cyclohex-1-enyl)phenoxy) carbonylamino)methyl)cyclohexyl)acetate**

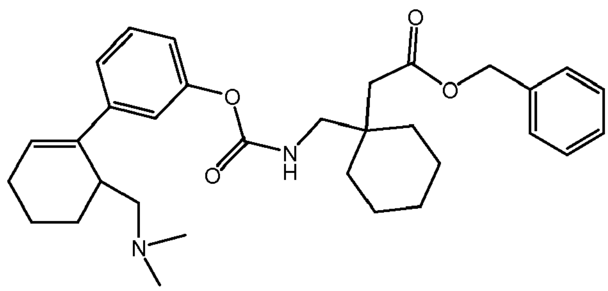

Purity HPLC: 91.9%

**$^1H$ -RMN (DMSO-$d_6$, 200 MHz):** 7.64 (t., 1H, NH); 7.40-7.10 (m, 7H); 7.01 (bs, 1H,); 6.92 (d., 1H); 5.96 (m, 1H, CH=C); 5.04 (s, 2H, $OCH_2$); 4.40-4.30 (bs, 1H, OH); 3.50-3.30 (m, 2H); 3.16 (bs, 2H, $NCH_2$); 2.37 (s, 2H, $CH_2CO_2$); 2.04 (s., 6H, $NCH_3$); 2.20-1.40 (m, 7H); 1.38 (bs, 10H,).

**$^{13}C$-RMN (DMSO-$d_6$, 50 MHz):** 171.16 (1C, C=O); 154.96 (1C, C=O); 151.25 (1C, C=C-O); 143.02 (1C, phenyl); 138.83 (1C, C=C); 136.20 (1C, C=CH, Benzyl); 128.99 (1C, C=C); 128.35 (2C, benzyl); 127.99 (2C, benzyl); 127.93 (1C, benzyl); 127.26 (1C, phenyl); 122.12 (1C, phenyl); 119.82 (1C, phenyl); 118.78 (1C, phenyl); 65.26 (1C, $CH_2O$); 61.37 (1C, $CH_2N$); 47.02 (1C, $CH_2NH$); 45.34 (2C, $NCH_3$); 40.75 (1C); 37.02 (1C); 32.82 (1C); 32.38 (2C); 25.59 (1C); 25.45 (1C); 24.66 (1C); 21.00 (2C); 17.15(1C).

**Example 24. phenyl 2-(1-(((3-(2-((dimethylamino)methyl)-1-hydroxy cyclohexyl)phenoxy)carbonylamino)methyl)cyclohexyl)acetate**

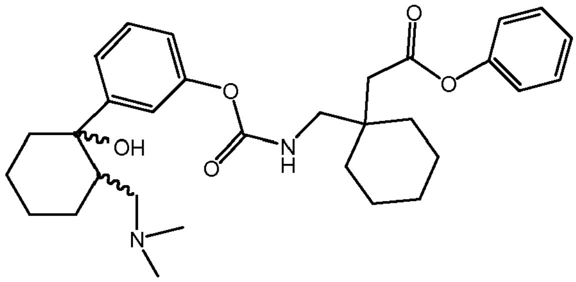

0.208g (2.2 mmol) of phenol, 0.51g (2.67 mmol) of EDAC (Fluka 03450) and 2mg (0.02mmol) of DMAP were added to 1.09g (2.45 mmol) of the product obtained in example 6 dissolved in 15 ml of $CH_2Cl_2$ and 0.5 ml of DMF. The mixture was stirred at room temperature for, concentrated to dryness and redissolved in 50 ml AcOEt. It was washed with 3x25 ml of water and the organic phase was dried, concentrated and cromatographed on silica gel. 0.46g of the product of the title were obtained eluting with 7/3 $CH_2Cl_2$/EtOH.

Purity HPLC: 87%

**$^1$H -RMN (DMSO-$d_6$, 200 MHz):** 7.73 (t., 1H, NH); 7.42-7.34 (m, 2H); 7.26-7.22 (m, 3H); 7.14-7.07 (m, 3H); 6.92-6.86 (m., 1H); 3.50-3.30 (m, 2H); 3.23 (d, J=6.6Hz, 2H, $NCH_2$); 2.58 (s, 2H, $CH_2CO_2$); 2.20-2.00 (s., 1H); 1.88 (s., 6H, $NCH_3$); 2.00-1.40 (m, 18H).

**$^{13}$C-RMN (DMSO-$d_6$, 50 MHz):** 169.97 (1C, C=O); 155.13 (1C, C=O); 151.37 (1C, C=C-O); 150.86 (1C, C=C-O); 150.35 (1C, phenyl); 129.37 (2C, phenyl); 128.28 (1C, phenyl); 125.67 (1C, phenyl); 121.85 (2C, phenyl); 121.35 (1C, phenyl); 118.93 (1C, phenyl); 118.26 (1C, phenyl); 74.39 (1C, COH); 60.31 (1C, $CH_2N$); 47.02 (1C, $CH_2NH$); 45.78 (2C, $NCH_3$); 43.03 (1C); 40.93 (1C); 39.90 (1C); 37.19 (1C); 32.54 (2C); 26.36 (1C); 25.49 (2C); 21.67 (1C); 21.01 (2C).

**Example 25. (R)-methyl 3-(((3-((2R,3S)-1-(dimethylamino)-2-methylpentan-3-yl) phenoxy)carbonylamino)methyl)-5-methylhexanoate**

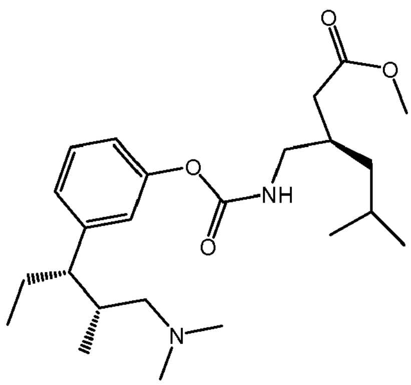

**25A) (S)-2-(2-methoxy-2-oxoethyl)-4-methylpentan-1-aminium chloride**

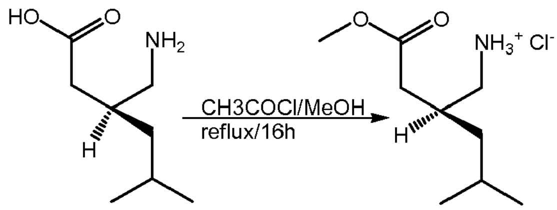


Following a procedure analogous to the one described in example 11A), but replacing 2-(1-(aminomethyl) cyclohexyl)acetic by (S)-3-(aminomethyl)-5-methyl-hexanoic acid the title product was obtained.

**$^1$H -RMN (DMSO-$d_6$, 200 MHz):** 3.57 (s., 3H, $OCH_3$); 2.71 (m, 2H, $NCH_2$); 2.60-2.22 (m, 2H, $CH_2CO_2$); 2.20-2.00 (m, 1H, CH); 1.60-1.45 (m, 1H, CH) 1.30-0.95 (m., 2H, $CH_2$); 0.81 (dd, 6H, $2CH_3$).

**$^{13}$C-RMN (DMSO-$d_6$, 50 MHz):** 171.46 (1C, C=O); 51.36 (1C, $OCH_3$); 41.82 (1C, $CH_2N$); 40.33 (1C, $CH_2CO_2$ 35.88 (1C); 30.94 (1C); 24.52 (1C); 22.47 (1C, $CH_3$); 22.41 (1C, $CH_3$).

**25B) (R)-methyl 3-(((3-((2R,3S)-1-(dimethylamino)-2-methylpentan-3-yl) phenoxy) carbonylamino)methyl)-5-methylhexanoate**

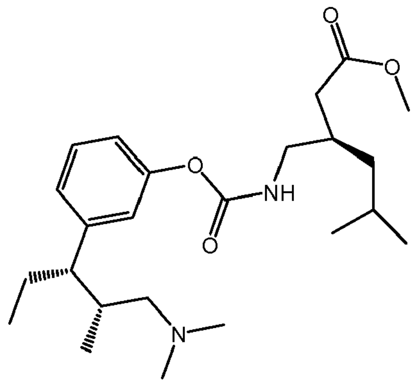

Following a procedure analogous to the one described in example 1B), but replacing (1-(2-methoxy-2-oxoethyl) cyclohexyl)methanaminium chloride by (S)-2-(2-methoxy-2-oxoethyl)-4-methylpentan-1-aminium chloride the product of the title was obtained.

Purity HPLC: 95.6%

**$^1$H -RMN (DMSO-$d_6$, 200 MHz):** 7.74 (t., 1H, NH); 7.27 (dd, 1H,); 6.92-6.80 (m., 3H); 3.58 (s., 3H, $OCH_3$); 3.50-3.20 (m, 2H); 3.20-2.70 (m, 2H, $NCH_2$); 2.40-2.20 (m, 2H, $CH_2CO_2$); 2.06 (s., 6H, $NCH_3$); 2.10-1.50 (m, 7H,); 1.30-1.00 (m., 1H); 1.04 (d.d., 3H, $CH_3$); 0.84(d.d., 6H, $2CH_3$); 0.65 (t, J=7.2Hz, 3H, $CH_3$)

**$^{13}$C-RMN (DMSO-$d_6$, 50 MHz):** 172.74 (1C, C=O); 154.63 (1C, C=O); 151.00 (1C, C=$\underline{C}$-O); 145.39 (1C, CH-$\underline{C}$=CH); 128.68 (1C, phenyl); 124.86 (1C, phenyl); 121.22 (1C, phenyl); 119.01 (1C, phenyl); 63.81 (1C, $CH_2N$); 51.17 (1C, $OCH_3$); 50.12 (1C, CH-Ph); 45.35 (2C, $NCH_3$); 43.98 (1C, $CH_2NH$); 40.59 (1C, $CH_2CO_2H$); 36.78 (1C); 35.85 (1C); 33.07 (1C); 24.68 (1C); 23.07 (1C); 22.71 (1C, $CH_3$); 22.41 (1C, $CH_3$); 15.53 (1C, $CH_3$); 12.17 (1C, $CH_3$).

**Examples 26 and 27**

Following a procedure analogous to the one described in example 25B), but replacing 3-((2R, 3S)-1-(dimethylamino)-2-methylpentan-3-yl)phenol by the corresponding products of formula (IIIa) the products of examples 26 and 27 have been obtained:

**Example 26. (R)-methyl 3-(((3-((1R,2R)-2-((dimethylamino)methyl)-1-hydroxy cyclohexyl)phenoxy)carbonylamino)methyl)-5-methylhexanoate**

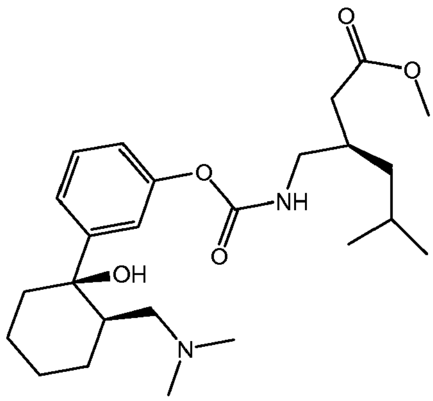

Purity HPLC: 98.9%

**$^1$H -RMN (DMSO-$d_6$, 200 MHz):** 7.74 (t., 1H, NH); 7.25 (bs, 2H,); 7.14 (s, 1H); 6.92-6.80 (m., 1H); 5.10, 4.90 (bs, 1H, OH); 3.58 (s., 3H, $OCH_3$); 3.50-3.20 (m, 2H); 3.20-2.80 (m, 2H, $NCH_2$); 2.40-2.20 (m, 2H, $CH_2CO_2$); 2.20-2.00 (m, 1H); 1.88 (s., 6H, $NCH_3$); 1.90-1.10 (m, 12H,); 0.84(d.d., 6H, $2CH_3$).

**$^{13}$C-RMN (DMSO-$d_6$, 50 MHz):** 172.76 (1C, C=O); 154.74 (1C, C=O); 151.42 (1C, C=C-O); 150.82 (1C, CH-C=CH); 128.30 (1C, phenyl); 121.36 (1C, phenyl); 118.96 (1C, phenyl); 118.29 (1C, phenyl); 74.41 (1C, COH-Ph); 60.32 (1C, $CH_2N$); 51.20 (1C, $OCH_3$); 45.86 (2C, $NCH_3$); 43.96 (1C, $CH_2NH$); 43.11 (1C); 40.91 (1C, $CH_2CO_2H$); 39.90 (1C); 36.78 (1C); 33.11 (1C); 26.37 (1C); 25.53 (1C); 24.68 (1C); 22.74 (1C, $CH_3$); 22.42 (1C, $CH_3$); 21.72 (C);

**Example 27. (R)-methyl 3-(((3-((S)-6-((dimethylamino)methyl)cyclohex-1-enyl) phenoxy)carbonylamino)methyl)-5-methylhexanoate**

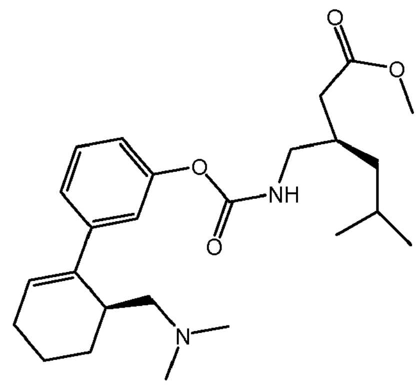

Purity HPLC: 97%

**$^1$H -RMN (DMSO-$d_6$, 200 MHz):** 7.78 (t., 1H, NH); 7.32-7.14 (m, 2H,); 7.02 (s, 1H); 6.95-6.91 (m., 1H); 5.99 (m, 1H, CH=C); 3.58 (s., 3H, $OCH_3$); 3.45-3.25 (m, 2H); 3.20-2.80 (m, 2H, $NCH_2$); 2.40-2.20 (m, 2H, $CH_2CO_2$); 2.16 (s., 6H, $NCH_3$); 2.10-1.40 (m, 9H,); 1.30-1.10 (m, 2H); 0.84(d.d., 6H, $2CH_3$).

**$^{13}$C-RMN (DMSO-$d_6$, 50 MHz):** 172.76 (1C, C=O); 154.64 (1C, C=O); 151.21 (1C, C=C-O); 143.00 (1C, CH-C=CH); 138.77 (1C, C=CH); 129.04 (1C, C=CH); 127.35 (1C, phenyl); 122.18 (1C, phenyl); 119.83 (1C, phenyl); 118.82 (1C, phenyl); 61.31 (1C, $CH_2N$); 51.19 (1C, $OCH_3$); 45.35 (2C, $NCH_3$); 43.99 (1C, $CH_2NH$); 40.90 (1C, $CH_2CO_2H$); 39.90 (1C); 36.79 (1C); 33.08 (1C); 32.76 (1C); 25.59 (1C); 24.68 (1C); 22.73 (1C, $CH_3$); 22.41 (1C, $CH_3$); 17.14 (1 C);

**Example 28. (R)-allyl 3-(((3-((2R,3S)-1-(dimethylamino)-2-methylpentan-3-yl) phenoxy)carbonylamino)methyl)-5-methylhexanoate**

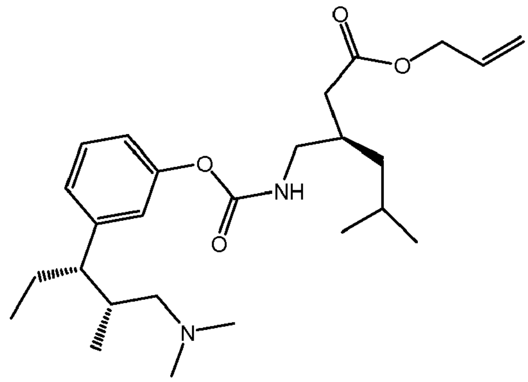

**28A) (S)-2-(2-(allyloxy)-2-oxoethyl)-4-methylpentan-1-aminium chloride**

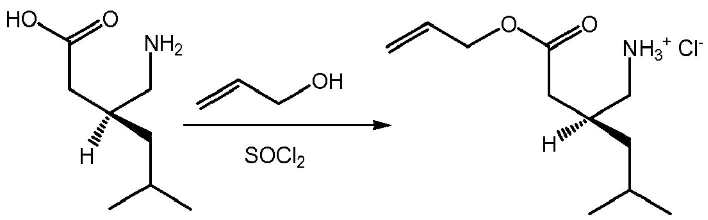


Following a procedure analogous to the one described in example 11A), but replacing 2-(1-(aminomethyl) cyclohexyl)acetic by (S)-3-(aminomethyl)-5-methylhexanoic acid the title product was obtained.

**$^1$H -RMN (DMSO-$d_6$, 200 MHz):** 8.20 (bs,3N, $NH_3^+$); 6.00-5.82 (m, 1H, CH=$CH_2$); 5.33-5.16 (m, 2H, CH=$CH_2$); 4.53 (dd., 2H, $OCH_2$); 2.72 (d, 2H, $NCH_2$); 2.66-2.25 (m, 2H, $CH_2CO_2$); 2.20-2.10 (m, 1H, CH); 1.65-1.45 (m, 1H, CH) 1.30-1.05 (m., 2H, $CH_2$); 0.81(dd, 6H, $2CH_3$).

**$^{13}$C-RMN (DMSO-$d_6$, 50 MHz):** 171.49 (1C, C=O); 132.61 (1C, C=C); 117.91 (1C, C=C); 64.46 (1C, $OCH_2$); 41.81 (1C, $CH_2N$); 40.29 (1C, $CH_2CO_2H$); 35.98 (1C); 30.96 (1C); 24.50 (1C); 22.50 (1C, $CH_3$); 22.38 (1C, $CH_3$).

**28B) (R)-allyl 3-(((3-((2R,3S)-1-(dimethylamino)-2-methylpentan-3-yl) phenoxy)carbonylamino)methyl)-5-methylhexanoate (LA13067)**

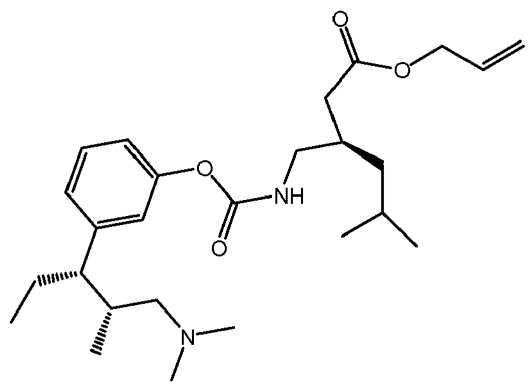

Following a procedure analogous to the one described in example 1B), but replacing (1-(2-methoxy-2-oxoethyl) cyclohexyl)methanaminium chloride by (S)-2-(2-(allyloxy)-2-oxoethyl)-4-methylpentan-1-aminium chloride (obtained in 28A) the product of the title was obtained.

Purity HPLC: 95.9%

**$^1$H -RMN (DMSO-$d_6$, 200 MHz):** 7.74 (t., 1H, NH); 7.27 (dd, 1H,); 6.98-6.84 (m., 3H); 6.00-5.80 (m, 1H, CH=$CH_2$); 5.33-5.16 (m, 2H, CH=$CH_2$); 4.54 (dd., 2H, $OCH_2$); 3.50-3.30 (m, 2H); 3.20-2.80 (m, 2H, $NCH_2$); 2.40-2.10 (m, 2H, $CH_2CO_2$); 2.06 (s., 6H, $NCH_3$); 2.00-1.50 (m, 7H,); 1.30-1.00 (m., 1H); 1.04 (d.d., 3H, $CH_3$); 0.84(d.d., 6H, $2CH_3$); 0.65 (t, J=7,2Hz, 3H, $CH_3$)

**$^{13}$C-RMN (DMSO-$d_6$, 50 MHz):** 171.94 (1C, C=O); 154.66 (1C, C=O); 151.02 (1C, C=C-O); 145.40 (1C, CH-C=CH); 132.74 (1C, C=C); 128.68 (1C, phenyl); 124.87 (1C, phenyl); 121.23 (1C, phenyl); 119.01 (1C, phenyl); 117.91 (1C, C=C); 64.30 (1C, $OCH_2$); 63.83 (1C, $CH_2N$); 50.15 (1C, CH-Ph); 45.35 (2C, $NCH_3$); 43.98 (1C, $CH_2NH$); 40.87 (1C, $CH_2CO_2H$); 36.90 (1C); 35.88 (1C); 33.10 (1C); 24.69 (1C); 23.08 (1C); 22.76 (1C, $CH_3$); 22.40 (1C, $CH_3$); 15.54 (1C, $CH_3$); 12.17 (1C, $CH_3$).

**Examples 29 and 30**

Following a procedure analogous to the one described in example 28B), but replacing 3-((2R, 3S)-1-(dimethylamino)-2-methylpentan-3-yl)phenol by the corresponding products of formula (IIIa) the products of the example 29 and 30 have been obtained:

**Example 29. (R)-allyl 3-(((3-((1R,2R)-2-((dimethylamino)methyl)-1-hydroxy cyclohexyl)phenoxy)carbonylamino)methyl)-5-methylhexanoate**

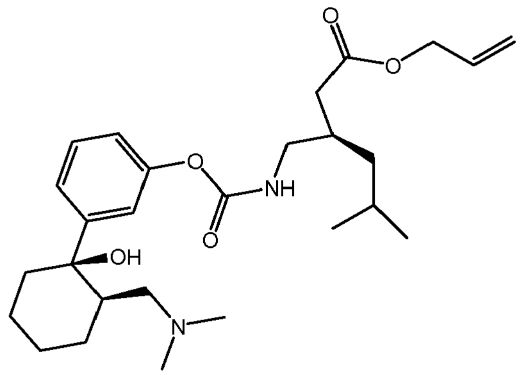

Purity HPLC: 94.8%

**$^1$H -RMN (DMSO-$d_6$, 200 MHz):** 7.74 (t., 1H, NH); 7.27 (bs, 2H,); 7.13 (s, 1H); 6.92-6.80 (m., 1H); 6.00-5.80 (m, 1H, CH=$CH_2$); 5.33-5.16 (m, 2H, CH=$CH_2$); 5.10, 4.90 (bs, 1H, OH); 4.53 (d., 2H, $OCH_2$); 3.50-3.30 (m, 2H); 3.20-2.80 (m, 2H, $NCH_2$); 2.40-2.20 (m, 2H, $CH_2CO_2$); 2.20-2.00 (m, 1H); 1.88 (s., 6H, $NCH_3$); 1.90-1.10 (m, 12H,); 0.83(d.d., 6H, $2CH_3$).

**$^{13}$C-RMN (DMSO-$d_6$, 50 MHz):** 171.96 (1C, C=O); 154.77 (1C, C=O); 151.42 (1C, C=C-O); 150.85 (1C, CH-C=CH); 132.74 (1C, C=C); 128.32 (1C, phenyl); 121.38 (1C, phenyl); 118.99 (1C, phenyl); 118.31 (1C, phenyl); 117.75 (1C, C=C); 74.44 (1C, COH-Ph); 64.33 (1C, $OCH_2$); 60.34 (1C, $CH_2N$); 45.86 (2C, $NCH_3$); 43.95 (1C, $CH_2NH$); 43.13 (1C); 40.99 (1C, $CH_2CO_2H$); 39.90 (1C); 36.90 (1C); 33.14 (1C); 26.39 (1 C); 25.55 (1C); 24.71 (1C); 22.79 (1C, $CH_3$); 22.42 (1C, $CH_3$); 21.72 (1C).

**Example 30. (3R)-allyl 3-(((3-(6-((dimethylamino)methyl)cyclohex-1-enyl) phenoxy)carbonylamino)methyl)-5-methylhexanoate**

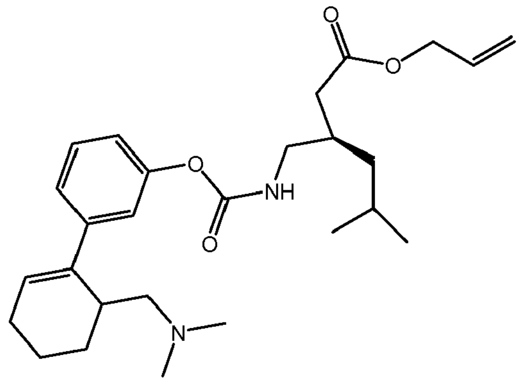

Purity HPLC: 99%

**$^1$H -RMN (DMSO-$d_6$, 200 MHz):** 7.78 (t., 1H, NH); 7.32-7.14 (m, 2H,); 7.01 (s, 1H); 6.95-6.91 (m., 1H); 5.99 (m, 1H, CH=C); 6.00-5.80 (m, 1H, CH=$CH_2$); 5.33-5.16 (m, 2H, CH=$CH_2$); 4.53 (d., 2H, $OCH_2$); 3.45-3.25 (m, 2H); 3.20-2.80 (m, 2H, $NCH_2$); 2.40-2.20 (m, 2H, $CH_2CO_2$); 2.07 (s., 6H, $NCH_3$); 2.10-1.40 (m, 9H,); 1.30-1.10 (m, 2H); 0.84 (d.d., 6H, $2CH_3$).

**$^{13}$C-RMN (DMSO-$d_6$, 50 MHz):** 171.95 (1C, C=O); 154.67 (1C, C=O); 151.23 (1C, C=C-O); 143.02 (1C, CH-C=CH); 138.77 (1C, C=CH); 132.75 (1C, C=C); 129.04 (1C, C=CH); 127.37 (1C, phenyl); 122.19 (1C, phenyl); 119.84 (1C, phenyl); 118.84 (1C, phenyl); 117.72 (1C, C=C); 64.32 (1C, $OCH_2$); 61.31 (1C, $CH_2N$); 45.35 (2C, $NCH_3$); 43.97 (1C, $CH_2NH$); 40.87 (1C, $CH_2CO_2H$); 39.90 (1C); 36.90 (1C); 33.11 (1C); 32.79 (1C); 25.60 (1C); 24.70 (1C); 22.78 (1C, $CH_3$); 22.39 (1C, $CH_3$); 17.16 (1C);

## B) PHARMACOLOGY

### Description of the pharmacological processes

### Analgesic Activity Test

The pharmacological activity of the products of the present invention was tested *in vivo* in formalin test model, which is known to evaluate neuropathic and inflammatory pain in animals.

### The Formalin Test: Characteristics and Usefulness of the Model

Sawynok, Jana; Liu, Xue Jun. Reviews in Analgesia, Volume 7, Number 2, 2003, pp. 145-163

The formalin test was introduced as a model of tonic pain in 1977, and has since been used extensively in rats and mice. In rats, formalin generates an initial phase of activity (5-10 min, phase 1), a quiescent interphase (5-10 min), and a second phase of activity (lasting 60-90 min, phase 2), and this is seen with spontaneous behaviors, firing of afferent neurons, and activity in dorsal horn neurons. Both active phases involve ongoing peripheral afferent neural activity; inflammation contributes to phase 2 activity and the interphase results from active inhibition. Responses are concentration dependent between 0.25% and 2.5%, plateau from 2.5% to 5%, and can decline at higher concentrations. Formalin also results in tissue edema, and this is longer lasting. Responses to formalin up to 2.5% are predominantly neurogenic, while at higher concentrations, responses involve a further prominent inflammatory component. Within the spinal cord, formalin increases c-Fos expression in neurons and causes activation of microglia, and these may contribute more prominently to longer term changes. Acute responses (to 90 min) may represent a model of ongoing acute pain involving inflammation and aspects of central sensitization, while longer term responses (days, weeks) may represent a model of changes involved in persistent pathological pain.

### Experimental Procedure

Male Swiss mice (weight range, 25-30 g) were used (n=6-8 per group).Mice were fasted for 3 hours before the administration of oral treatment with free access to drinking water before the experiment. All experiments were conducted in accordance with the "Guidelines on Ethical Standards for Investigation of Experimental Pain in Animals

Mice were housed in clear plastic chambers with a mirror placed at a 45° angle to allow an unobstructed view of the paws. Mice were injected into the dorsal surface of hind paw with 20 μl of dilute formalin (5%) using a 30-gauge needle. Treatments were administrated p.o. 10 min before formalin injection.

Animals were observed for nociceptive behavior immediately after formalin injection during 60 min. Nociceptive behavior was quantified measuring time spent licking the injected paw after injection.

For evaluation of antinociceptive effect of a potential drug for neuropathic pain, the quantization of the second phase of the test (15-60 min) is the most important. Controls animals were observed under identical conditions without receiving treatments before formalin injection.

For each dose of compound given the % inhibition of licking vs. control animals were calculated using the following equation:

$$\% \text{ inhibition vs control} = 100 \times (\text{time spent licking control} - \text{time spent licking treatment}) / \text{time spent licking control}$$

The % inhibition vs control was measured for each compound at different doses and the $ED_{50}$ values (dose of compound that produced 50% inhibition) were calculated using the method of Litchfield and Wilcoxon. At the end of the experiments, mice were humanely euthanized with a dose of sodium pentobarbital (150 mg/kg i.p.).

| PRODUCT | FORMALIN TEST Phase II ($ED_{50}$ p.o) µmol/kg |
|---|---|
| Tramadol hydrochloride | 138.7 |
| Tramadol Enantiomer R,R | 126.6 |
| Tramadol Enantiomer S,S | 233.3 |
| Product of example 1 | 32.9 |
| Product of example 2 | 29.5 |
| Product of example 3 | 24.6 |
| Product of example 4 | 35.3 |
| Product of example 5 | 29.6 |
| Product of example 6 | 59.3 |
| Product of example 7 | 36.7 |
| Product of example 8 | 84.9 |
| Product of example 9 | 73.3 |
| Product of example 10 | 31.7 |
| Product of example 11 | 62.6 |
| Product of example 12 | 56.5 |
| Product of example 13 | 44.1 |
| Product of example 14 | 60.7 |
| Product of example 15 | 54.9 |
| Product of example 16 | n.d. |
| Product of example 17 | 61.3 |
| Product of example 18 | 42.5 |
| Product of example 19 | 60.2 |
| Product of example 20 | 55.6 |
| Product of example 21 | 67.4 |
| Product of example 22 | 52.7 |
| Product of example 23 | n.d. |
| Product of example 24 | n.d. |

(continued)

| PRODUCT | FORMALIN TEST Phase II ($ED_{50}$ p.o) μmol/kg |
|---|---|
| Product of example 25 | 84.1 |
| Product of example 26 | 63.3 |
| Product of example 27 | 83.5 |
| Product of example 28 | n.d |
| Product of example 29 | 74.9 |
| Product of example 30 | n.d. |
| n.d.: not determined | |

**Claims**

1. A compound of formula (I), or pharmaceutically acceptable, stereoisomers, salts or solvates thereof

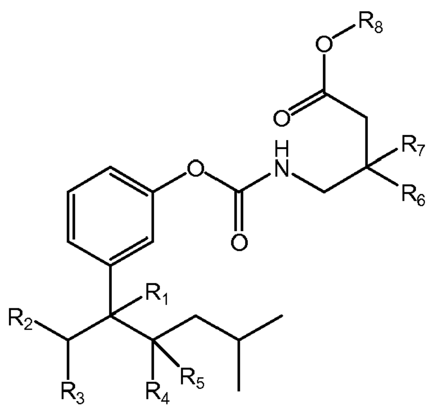

(I)

wherein
$R_1$ is selected from the group consisting of -H, and -OH;
$R_2$ is -H;
$R_5$ is -H;
or $R_1$ and $R_2$ together form a double bond and $R_5$ is -H
or $R_1$ and $R_5$ together form a double bond and $R_2$ is -H;
$R_3$ is a $C_1$-$C_6$ alkyl group;
$R_4$ is a $C_1$-$C_6$ alkyl group;
or $R_3$ and $R_4$ together form a $C_2$-$C_4$ alkylidene group;
$R_6$ is selected from the group consisting of -H and $C_1$-$C_6$ alkyl group;
$R_7$ is selected from the group consisting of -H and $C_1$-$C_6$ alkyl group;
or $R_6$ and $R_7$ together form a $C_4$-$C_6$ alkylidene group;
$R_8$ is selected from the group consisting of -H, $C_1$-$C_6$ alkyl, $C_2$-$C_6$ alkenyl unsubstituted $C_6$-$C_{14}$ aryl, substituted $C_6$-$C_{14}$ aryl and benzyl.

2. A compound according to claim 1, wherein $R_6$ is hydrogen and $R_7$ is a $C_1$-$C_6$ alkyl group.

3. A compound according to claim 2, wherein $R_7$ is a branched $C_1$-$C_6$ alkyl group.

4. A compound according to claim 1, wherein $R_6$ and $R_7$ together form a $C_4$-$C_6$ alkylidene group.

5. A compound according to claim 4, wherein $R_6$ and $R_7$ form a cyclohexyl group together with the carbon atom to

which they are attached.

**6.** A compound according to any one of claims 1-5, wherein $R_8$ is a hydrogen atom or a $C_1$-$C_3$ alkyl group.

**7.** A compound according to claim 6 wherein $R_8$ is a methyl group.

**8.** A compound according to any one of claims 1-7, wherein $R_1$ is hydroxyl.

**9.** A compound according to any one of claims 1-7 of formula (IA), or pharmaceutically acceptable, stereoisomers, salts or solvates thereof

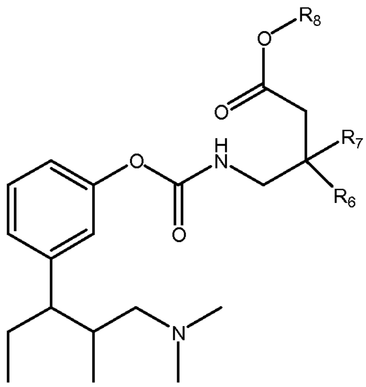

(IA)

wherein $R_6$, $R_7$ and $R_8$ are as defined in the previous claims.

**10.** - A compound according to claims 1-8 of formula (IB), or pharmaceutically acceptable, stereoisomers, salts or solvates thereof

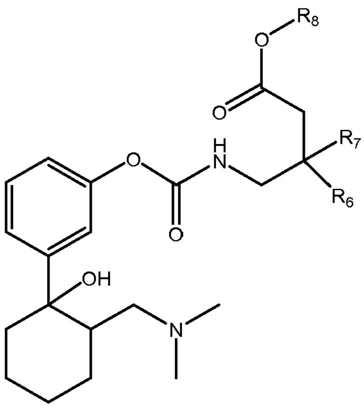

(IB)

wherein $R_6$, $R_7$ and $R_8$ are as defined in the previous claims.

**11.** - A compound according to claims 1-7 of formula (IC), or pharmaceutically acceptable, stereoisomers, salts or solvates thereof

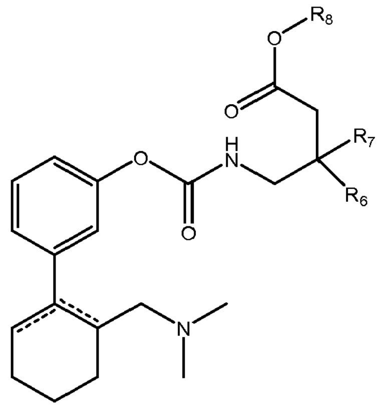

(IC)

wherein $R_6$, $R_7$ and $R_8$ are as defined in the previous claims, and one of the dotted lines may represent a double bond.

**12.** - A compound according to claim 5 of formula (IE)

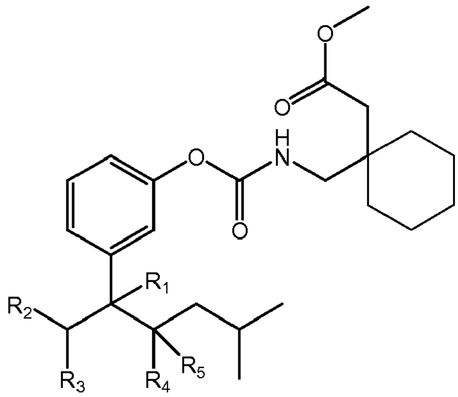

(IE)

wherein $R_1$ to $R_5$ are as defined in claim 1.

**13.** - A compound as defined in any of claims 1-12 for use as a medicament.

**14.** - Pharmaceutical composition comprising a compound as defined in any of claims 1-12 and a pharmaceutically acceptable carrier.

**15.** - Compound as defined in any of claims 1-12 for use in the treatment of pain.

**16.** - Compound for use as defined in claim 15, wherein pain is chronic pain.

**17.** - Compound for use as defined in claim 15 or 16, wherein pain is neurophatic pain.

Europäisches Patentamt
European Patent Office
Office européen des brevets

# EUROPEAN SEARCH REPORT

Application Number
EP 11 38 2184

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2010/227921 A1 (FRANKLIN RICHARD [GB] ET AL) 9 September 2010 (2010-09-09)<br>* the whole document *<br>----- | 1-17 | INV.<br>C07C271/54<br>A61K31/27<br>A61P25/04 |
| | | | TECHNICAL FIELDS SEARCHED (IPC) |
| | | | C07C |

The present search report has been drawn up for all claims

1

| Place of search | Date of completion of the search | Examiner |
|---|---|---|
| Munich | 2 January 2012 | Cooper, Simon |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 38 2184

This annex lists the patent family members relating to the patent documents cited in the above-mentioned European search report.
The members are as contained in the European Patent Office EDP file on
The European Patent Office is in no way liable for these particulars which are merely given for the purpose of information.

02-01-2012

| Patent document cited in search report | Publication date | Patent family member(s) | Publication date |
|---|---|---|---|
| US 2010227921 A1 | 09-09-2010 | US 2010227921 A1 | 09-09-2010 |
| | | WO 2010100477 A2 | 10-09-2010 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009067703 A
- WO 2009021058 A
- US 6562865 B
- EP 1251120 A
- WO 0027799 A1
- EP 0753506 A
- US 2005143355 A
- WO 2010100477 A2
- CN 101845002 A
- EP 0693475 A1
- EP 0753506 A1
- WO 2004108658 A1
- NL 6610022
- ES 2138271
- US 3564100 A
- EP 0786450 A
- EP 693475 A
- WO 2008012047 A
- US 20090005352 A1
- WO 2008060572 A
- US 4024175 A


### Non-patent literature cited in the description


- **E.E. CODD et al.** *Pain,* 2008, 254-262
- **V. GRANADOS-SOTO et al.** *Pharmacology,* 2005, vol. 74, 200-208
- **FLICK et al.** *Arzneim. Forsch/Drug Res.,* 1978, vol. 28 (I), 107-113
- **GRAHAM R. et al.** *Org. Proc. Res. Dev.,* 2002, vol. 6 (5), 729-737
- Advanced Organic Chemistry: reactions, mechanisms and structures. Wiley-interscience, 484-488
- IASP, Classification of chronic pain. IASP Press, 2002, 210-212
- **E.W. MARTIN.** *Remington's Pharmaceutical Sciences*
- **C. FAULÍ ; TRILLO.** Tratado de Farmacia Galénica. 1993
- **SAWYNOK, JANA ; LIU, XUE.** *Reviews in Analgesia,* June 2003, vol. 7 (2), 145-163